Europäisches Patentamt

European Patent Office

Office européen des brevets

(19)

(11) Publication number: **0 035 265**
**B1**

(12) # EUROPEAN PATENT SPECIFICATION

(45) Date of publication of patent specification: **02.01.86**

(51) Int. Cl.⁴: **A 61 K 49/02**

(21) Application number: **81101473.7**

(22) Date of filing: **02.03.81**

(54) **Agent for tumor localization and therapy with labeled antibodies and antibody fragments.**

(30) Priority: **03.03.80 US 126261**
**03.03.80 US 126262**
**03.03.80 US 126263**

(43) Date of publication of application:
**09.09.81 Bulletin 81/36**

(45) Publication of the grant of the patent:
**02.01.86 Bulletin 86/01**

(84) Designated Contracting States:
**AT BE CH DE FR GB IT LI LU NL SE**

(56) References cited:
**US-A-3 927 193**

**BIOLOGICAL ABSTRACTS, vol. 58, 15th July
1974, ref. no. 8934 U.S.A. A.E. REIF et al.: "Trial
of radio-labeled antibody localization in
metastases of a patient with a tumor
containing carcinoembryonic antigen (CEA)**

**BIOLOGICAL ABSTRACTS, vol. 59, 15th April
1975, ref. no. 44491 U.S.A. HSIA-FU CHAO et
al.: "Selective uptake of specifically purified
antibodies to carcinoembryonic antigen of
human adenocarcinoma"**

**The file contains technical information
submitted after the application was filed and
not included in this specification**

(73) Proprietor: **Goldenberg, Milton David**
**11 Denman Court**
**Short Hills New Jersey 07078 (US)**

(72) Inventor: **Goldenberg, Milton David**
**11 Denman Court**
**Short Hills New Jersey 07078 (US)**

(74) Representative: **Rasper, Joachim, Dr.**
**Bierstadter Höhe 22**
**D-6200 Wiesbaden (DE)**

(56) References cited:

**BIOLOGICAL ABSTRACTS, vol. 61, no. 12, 1976,
ref. no. 67779 U.S.A. E.B. SILBERSTEIN:
"Cancer diagnosis: The role of tumor-imaging
radiopharmaceuticals"**

**NATURE, vol. 248, 19th April 1974, pages
704-706 J.-P. MACH et al.: "In vivo localisation
of radiolabelled antibodies to
carcinoembryonic antigen in human colon
carcinoma grafted into nude mice"**

**BIOLOGICAL ABSTRACTS, vol. 67, ref. no.
16852, no. 3, 1953 U.S.A. D.M. GOLDENBERG
et al.: "Use of radiolabeled antibodies to
carcino-embryonic antigen for the detection
and localization of diverse cancers by external
photoscanning"**

Courier Press, Leamington Spa, England.

EP 0 035 265 B1

## Description

Background of the invention

It is known that radiolabeled antibodies to carcinoembryonic antigen (CEA) can be used to localize tumors. U.S. Patent 3,927,193, to Hansen et al, discloses such a method, but provides examples of its use only in animals. The method described in this patent does not explain how tumors may be visualized in a situation where radioactivity is also present in other sites of the body, such as blood, other body fluids and certain tissues, particularly heart and liver, which can prevent precise discrimination of the radioactivity associated with the sites of tumor. Early clinical studies reported by Reif et al. *J. Surg. Oncol., 6,* 133 (1974) and Mach et al, *Europ. J. Cancer, Suppl. 1,* 113 (1978) failed to show tumor localization in humans with radioactive anti-CEA antibodies.

Goldenberg et al, in an article in the *New England Journal of Medicine, 298,* 1384 (1978), reported success in clinical trials of tumor detection and localization by scintillation scanning of patients receiving radiolabeled antibodies to CEA. In that reference, it was noted that there was a problem in both animal and human studies in distinguishing specific radioantibody activity from bloodpool background activity, and that special scanner subtraction techniques with other radionuclides were considered essential for unequivocal tumor localization using this method. The antibody preparation used in the reference was 70% immunoreactive with CEA. The reference further notes that the absence of CEA in normal hamster tissues precludes extrapolation to man, in whom the antigen usually circulates in increased levels in patients with cancer, and is present in lesser quantities in certain normal tissues. The subtraction technique used to permit localization using this scintigraphic method involved injection of Te-99m-pertechnetate and Tc-99m-labeled human serum albumin prior to each imaging scan. The data obtained were stored in a minicomputer capable of generating digital images of the labeled antibody alone, the Tc-99m labeled species together, and sums and differences of these various values.

CEA is considered to be primarily a cell-surface antigen, as reported by Heyderman, *Scand. J. Immunol., 8., Suppl. 8,* 119 (1978), and many others. It had been thought that tumor localization in man required antibodies which were specific to antigens located on the surface of the tumor cell, by Spar, *Seminars In Nucl. Med., 6,* 379 (1976) and Emrich, *Deutsche Med. Woch. Schr., 104,* 153 (1979). It is known that both human chorionic gonadotropin (HCG) and alphafetoprotein (AFP) are cytoplasmic intracellular tumor-associated substances, Heyderman, *supra,* and by Lee et al, Guillouzo et al, Albrechtsen et al and Ruoslahti et al, in *Scand. J. Immunol., 8, Supp. 8,* pp. 485ff, 289ff, 165ff and 3ff, respectively (1978).

Quinones et al, *J. Nucl. Med., 12,* 69 (1971) demonstrated that a human choriocarcinoma grown in hamsters could show a 2.8-fold increased uptake of radiolabeled anti-HCG antibody in the tumor as compared to that in the animal's liver. However, control studies with labeled normal IgG showed similar increased tumor uptake through the second day postinjection. Moreover, the authors specifically stated that the difference in uptake of radioantibody over labeled normal IgG found by tissue analysis in days 3 and 4 was not observed by total body photoscanning. Hirai et al, *Abstracts 6th Int. Res. Group for Carcinoembryonic Proteins,* Marburg/Lahn, Fed. Rep. of Germany, 17—21 Sept., 1978, reported that administration of radiolabeled anti-AFP antibodies to rodents with transplanted human hepatoma, and with rat and human yolk sac tumors, revealed no "homing in" of the antibody in the tumor tissues.

Radioactive labeled fragments obtained by cleavage of antibodies specific to cardiac myosin have been used to determine the location and size of myocardial infarcts, in U.S. Patent 4,036,945 to Haber.

Tumor rariotherapy using antibodies has been suggested by many, and an indication of its success in a single multimodal therapeutic clinical use is reported by Ettinger, Cancer Treat. Rep., *63,* 131 (1979). The use of boron-labeled antibodies in therapy is reported by Hawthorne et al., *J. Med. Chem., 15,* 449 (1972), but the combined incorporation of boron for therapy and a radioisotope for localization is not suggested.

Even the recent and successful tumor localization and detection process of Goldenberg et al. *supra,* has certain disadvantages which limit its resolution, its efficiency and its practicability. U.S. Patent 3,927,193 teaches that the anti-CEA antibody should not be labeled to a degree which might interfere with its specificity, a limitation which was not questioned in the later references discussed above. However, this limits the resolution of the method and requires larger quantities of antibody for image detection.

The labeled antibodies are very large molecules which may also carry cross-reactive antigenic determinants, and it is quite difficult to reduce cross-reactivity below 15% and to achieve specificity for a particular antigen of higher than 70%. The subtraction technique used by Goldenberg et al involves the use of a different radionuclide attached to a carrier having kinetics of transport and distribution different from the labeled specific antibody. In addition, the background-compensating material must be injected prior to each photoscan, which exposes the patient to increased levels of radioactivity and discomfort. A further limitation of the prior art methods is that antibody molecules cannot pass the blood-brain barrier, which means that intravenously injected antibodies cannot be used to localize intracranial tumors.

A need continues to exist for methods of tumor detection and localization which are not confined to the use of antibodies to cell-surface antigens, which do not require repeated injection of background-compensating material for a subtraction technique, which are adaptable to both diagnosis and therapy, which have high reliabilty and high resolution, which ideally are capable of detecting and locating more than one type of tumor or tumor cells using a single injection, and which avoid other disadvantages of the prior art methods.

Objects of the invention

Accordingly, it is an object of the present invention to provide injectable compositions comprising radiolabeled antibodies or antibody fragments, and independently detectable radiolabeled reference compounds, for use in methods of tumor localization and detection which achieve high resolution without the necessity of repeated injection of other radioactive material for computer-assisted subtraction of background activity.

A further object of the present invention is to provide a means of tumor localization and detection which achieves high resolution and which uses antibodies to intracellular tumor-associated marker substances.

Another object of the present invention is to provide radiolabeled antibodies, antibody fragments and injectable compositions comprising them, having a combination of high specific activity and high specificity for tumor markers, for use in improving the resolution of scintigraphic tumor localization and detection methods, and for tumor radiotherapy.

A still further object of the invention is to provide multivalent antibody fragments containing in chemical combination fragments having specificity to more than one type of tumor-associated antigen or marker.

Yet another object of this invention is to provide radiolabeled antibodies or antibody fragments which further contain boron addends, for use in a method of tumor therapy wherein thermal neutrons excite the boron-10 isotope-containing antibody or antibody fragment which has been localized by detection of an attached ratioisotope label.

Upon further study of the specification and appended claims, further objects and advantages of this invention will become apparent to those skilled in the art.

Summary of the invention

The foregoing objects are achieved by providing a F(ab')₂ hybrid formed from two chemically joined Fab' fragments, said fragments being a first marker-specific Fab' fragment obtained by cleavage of an antibody specific to a first tumor-associated marker and a second, different marker-specific Fab' fragment obtained by cleavage of a second antibody specific to the same or different tumor-associated marker, said hybrid being radiolabeled with a pharmacologically inert radioisotope capable of detection using a photoscanning device.

The invention further provides an antibody or antibody fragment which is specific to a marker substance produced by or associated with a tumor, said antibody or antibody fragment being radiolabeled with a pharmacologically inert radioisotope capable of detection with a photoscanning device, said labeled antibody or antibody fragment further containing in chemical combination an addend containing at least five atoms of boron with at least a natural abundance of Boron-10 isotope.

In addition, the invention provides an injectable composition, comprising (a) an antibody specific to a tumor cell-surface marker substance and radiolabeled with a pharmacologically inert radioisotope capable of detection using a photoscanning device, (b) normal immunoglobulin from the same or different species as that used to prepare said specific antibody, said normal immunoglobulin being radiolabeled with a different pharmacologically inert radioisotope from that used to label the specific antibody and emitting at an energy capable of independent detection using said photoscanning device, said labeled normal immunoglobulin having substantially the same kinetics of binding, distribution and metabolism as the labeled specific antibody, and (c) a pharmaceutically acceptable injection vehicle; for use in a method for determining the location of a tumor which produces or is associated with a cell-surface marker substance, using a subtraction technique.

An injectable composition is also provided, comprising (a) a substantially monospecific antibody, having a specific immunoreactivity of at least 70% to an intracellular marker substance produced by or associated with a tumor and a cross-reactivity to non-tumor associated antigens of less than 15%, said antibody being radiolabeled with a pharmacologically inert radioisotope capable of detection using a photoscanning device; (b) normal immunoglobulin from the same or different species as that used to prepare said specific antibody, said normal immunoglobulin being radiolabeled with a different pharmacologically inert radioisotope from that used to label the specific antibody and emitting at an energy capable of independent detection by said photoscanning device, said labeled normal immunoglobulin having substantially the same kinetics of binding, distribution and metabolism as the labeled specific antibody; and (c) a pharmaceutically acceptable injection vehicle; for use in a method for detecting and localizing a tumor which produces or is associated with an intracellular marker substance, using a subtraction technique.

The invention also provides an injectable composition, comprising (a) an antibody fragment specific to a tumor-associated cytoplasmic, intracellular or cell-surface marker, obtained by cleavage of an antibody specific to said marker substance, said fragment being radiolabeled with a pharmacologically inert radioisotope capable of detection using a photoscanning device; and (b) a pharmaceutically acceptable injection vehicle; for use in a method for detecting and localizing a tumor which produces or is associated with a cytoplasmic, intracellular or cell-surface marker substance.

Detailed discussion

The labeled antibodies, antibody fragments and injectable compositions of the invention are useful, *inter alia*, in methods for detecting and localizing tumors which either produce or are associated with the marker substances to which the antibodies or fragments are specifically immunoreactive, comprising injecting a human subject parenterally with the labeled antibody or fragment, optionally in combination with a labeled independently detectable reference compound, and scanning with a photoscanning device, optionally with subtraction of non-target background activity, to determine the site or sites of uptake of targeted antibody or fragment. They can also be used for tumor radiotherapy, including neutron-activated therapy by neutron irradiation of localized boron-containing antibodies.

The antibodies used in the present invention are specific to a variety of tumor-associated antigens or marker substances, while the marker-specific antibody fragments used in the method of the present invention are produced by cleavage of such specific antibodies. The markers may be substances produced by the tumor, substances which accumulate within tumor cells, whether in the cytoplasm, in the nucleus or in various organelles or subcellular structures, or substances which accumulate on or around tumor cells. They may be intracellular, cell surface or cytoplasmic markers. Among such tumor-associated markers are those disclosed by Herberman, "Immunodiagnosis of Cancer", in Fleisher, Ed., "The Clinical Biochemistry of Cancer", page 347 (Am. Assn. Clin. Chem. 1979) and in U.S. Patent 4,150,149 to Wolfsen et al.

Tumor-associated markers have been categorized by Herberman, *supra*, in a number of categories including oncofetal antigens, placental antigens, oncogenic or tumor virus-associated antigens, tissue-associated antigens, organ-associated antigens, ectopic hormones and normal antigens or variants thereof. Occasionally, a sub-unit of a tumor-associated marker is advantageously used to raise antibodies having higher tumor-specificity, e.g., the beta-subunit of human chorionic gonadotropin (HCG), which stimulates the production of antibodies having a greatly reduced cross-reactivity to non-tumor substances. Suitable such marker substances to which specific antibodies may be raised which are useful in the present invention include, but are not limited to, alphafetoprotein (AFP), human chorionic genadotropin (HCG) and/or its beta-subunit (HCG-beta), colon-specific antigen-p (CSAp), carcinoembryonic antigen (CEA), prostatic acid phosphatase, pancreatic oncofetal antigen, placental alkaline phosphatase, pregnancy $beta_1$-globulin, parathormone,

calcitonin, tissue polypeptide antigen, T-antigen, $beta_2$-microglobulin, mammary tumor-associated glycoproteins (MTGP), galactosyl transferase-II(GT-II), gp-52 viral-associated antigen, ovarian cystadenocarcinoma-associated antigen (OCAA), ovarian tumor-specific antigen (OCA), cervical cancer antigens (CA-58, CCA, TA-4), basic fetoprotein (BFP), terminal deoxynucleotidyl transferase (TdT), cytoplasmic melanoma-associated antigens, human astrocytoma-associated antigen (HAAA), common glioma antigen (CGA), glioembryonic antigen (GEA), glial fibrillary acidic protein (GFA), common meningioma antigen (CMA) and tumor angiogenesis factor (TAF).

Marker-specific antibodies may be produced by conventional methods well known in the art. Normally, an animal, preferably a rodent, a rabbit, or more preferably a goat or primate, is challenged with a tumor-associated marker substance, to which its immune system reacts by producing specific antibodies to these markers. The animal is bled, the immunoglobulin fraction of the blood is isolated, and the specific immunoglobulin isolated by a variety of conventional separation techniques, preferably including one or more affinity chromatography purification steps. Suitable such general methods for raising antibodies specific to tumor-associated marker substances are disclosed *inter alia* in "Immunodiagnosis of Cancer", Herberman et al, Eds. (Marcel Dekker, Inc., New York and Basel, 1979) and "Tumor Markers", Sell, Ed. (Humana Press, Clifton, N. J. 1980).

Antibodies specific to CEA may be produced by a variety of methods known in the art, as reported *inter alia* in Primus et al, *J. Immunol.*, *118*, 55 (1977); Goldenberg et al, *supra*; Primus et al, *Cancer Res.*, *37*, 1544 (1977); and Goldenberg et al, in "Immunodiagnosis of Cancer, Part I", Herberman et al, Eds., pages 265—304 (Marcel Dekker, Inc., New York & Basel, 1979).

Antibodies produced by the foregoing conventional techniques are normally mixtures of antibodies, a certain proportion of which are specific but generally containing a variable proportion of antibodies which are cross-reactive with non-tumor associated or other antigens. Antibodies purified by repeated affinity chromatography using bound antigens with which some components of the antibody mixture are cross-reactive, as well as passage through a column containing bound purified antigen, have a high specific immunoreactivity, often approaching or even exceeding 70%, and a cross-reactivity with non-tumor associated antigens or other antigens of less than 15%. These antibodies are considered substantially monospecific to the antigen to which they have been raised, and are preferably used in the present invention.

It is particularly advantageous to use antibodies having a high marker-specific immunoreactivity for tumor localization. High specificity means that a high proportion of the labeled antibody will be targeted at tumor sites and a small proportion will be distributed in a non-targeted manner. A smaller quantity of labeled antibody can therefore be used, reducing the subject's exposure to radiation, and the lower level of background radiation due to non-targeted antibody will improve resolution. This in turn means that smaller tumors may be detected that are often difficult or impossible to detect by any other procedure.

Highly specific monoclonal antibodies can be produced by hybridization techniques. Such antibodies normally require little or no purification and normally have a specific immunoreactivity of at least 75%, with specificities of more than 95% in certain cases. Such monoclonal, hybridoma-derived antibodies are also preferred for use in the present invention. In a preferred embodiment, monoclonal antibodies are produced by challenging a monkey with a purified tumor-associated marker, fusing antibody-producing monkey lymph or spleen cells with human or mouse myeloma cells to produce hybrid cells which are then isolated, cloned and selected for their ability to produce monoclonal antibodies specific to said marker substance.

Monoclonal antibodies from the immuno-globulin G(IgG) fraction are obtained by the present method, and are used to prepare the fragments used for tumor detection, localization and therapy according to this invention. The IgM monoclonal antibodies of Koprowski, U.S. Patent 4,172,124, are unsuitable for use in the present method.

It is known that antibody fragments may be produced by enzymatic cleavage of antibodies with pepsin to provide a 5S fragment denoted F(ab')$_2$. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using papain produces two monovalent Fab fragments and an Fc fragment directly. These methods are described *inter alia* in U.S. Patent 4,036,945 and references contained therein, and in Nisonoff et al, *Arch, Biochem. Biophys., 89,* 230 (1960); Porter, *Biochem. J., 73,* 119 (1959); and Edelman et al, in "Methods in Immunology and Immuno-chemistry", *Vol. 1,* 422 (Acad. Press, 1967).

Other methods of cleaving antibodies, such as further cleavage of Fab fragments or by the use of other enzymatic or chemical techniques may also be used. Only fragments which retain specificity to the tumor-associated marker against which their parent antibodies are raised are used in the present method.

Hybrid antibody fragments have been prepared by oxidative linkage of Fab' fragments resulting from reductive cleavage of different antibodies. A portion of these will contain fragments specific to both of the antigens to which the original antibodies were raised. This is advantageously carried out by mixing two different F(ab')$_2$ fragments produced by pepsin digestion of two different antibodies, reductive cleavage to form a mixture of Fab' fragments, followed by oxidative reformation of the disulfide linkages to produce a mixture of F(ab')$_2$ fragments including hybrid fragments containing an Fab' portion specific to each of the original antigens. Methods of preparing such hybrid antibody fragments are disclosed in Feteanu, "Labeled Antibodies in Biology and Medicine" pages 321—323 (McGraw-Hill Int. Bk. Co., New York et al, 1978); Nisonoff et al, *Arch Biochem. Biophys., 93,* 470 (1961); and Hammerling et al, *J. Exp. Med., 128,* 1461 (1968).

In the following discussion, the term "antibody" will embrace antibodies and antibody fragments as disclosed hereinabove, unless otherwise indicated.

Antibodies may be labeled by any of several techniques known to the art. A wide range of labeling techniques are disclosed in Feteanu, "Labeled Antibodies in Biology and Medicine", pages 214—309 (McGraw-Hill Int. Book Co., New York et al, 1978). The introduction of various metal radioisotopes may be accomplished according to the procedures of Wagner et al., *J. Nucl. Med., 20,* 428 (1979); Sundberg et al, *J. Med. Chem., 17,* 1304 (1974); and Saha et al, *J. Nucl. Med., 6,* 542 (1976). The foregoing are merely illustrative of the many methods of radiolabeling proteins known to the art.

Among the radioisotopes used, gamma-emitters, positron-emitters, x-ray-emitters and fluorescence-emitters are suitable for localization and/or therapy, while beta-emitters and alpha-emitters may also be used for therapy. Suitable radioisotopes for labeling antibodies include Iodine-131, Iodine-123, Iodine-126, Iodine-133, Bromine-77, Indium-111, Indium-113m, Gallium-67, Gallium-68, Ruthenium-95, Ruthenium-97, Ruthenium-103, Ruthenium-105, Mercury-197, Mercury-203, Rhenium-99m, Rhenium-105, Rhenium-101, Tellurium-121m, Tellurium-112m, Tellurium-125m, Thulium-165, Thulium-167, Thulium-168, Technetium-99m and Fluorine-18. The halogens can be used more or less interchangeably as labels since halogen-labeled antibodies or fragments and/or normal immunoglobulins or the corresponding fragments would have substantially the same kinetics and distribution and similar metabolism.

A preferred technique for labeling antibodies involves labeling with either Iodine-131 (I-131) or Iodine-123 (I-123) using an oxidative procedure wherein a mixture of radioactive potassium or sodium iodide and the antibody is treated with chloramine-T, e.g., as reported by Greenwood et al, *Biochem. J., 89,* 114 (1963) and modified by McConahey et al, *Int. Arch. Allergy Appl. Immunol., 29,* 185 (1969). This results in direct substitution of iodine atoms for hydrogen atoms on the antibody molecule, presumably on tyrosine residues, possibly also on tryptophan and even on phenylalanine residues, depending on the proportions of reagents and the reaction conditions.

In general, it is desirable to introduce as high a proportion of radiolabel as possible into the antibody molecule without destroying its immunospecificity. While the vast majority of investigators had considered that introduction by direct substitution of more than from 1.5 to 2 iodine atoms per antibody molecule is disadvantageous, it has now been found that the introduction by direct substitution of at least 2.5 and preferably an average of from 5 to 10 iodine atoms per antibody molecule is advantageous, especially where the antibody is highly marker-specific prior to labeling. In this case, even a reduction of the antibody specificity of from 5 to 33% as a consequence of high labeling is outweighed by the advantage of high activity, permitting the use of substantially smaller quantities of labeled antibody. As noted above, the use of highly specific antibodies of high activity results in efficient localization and increased resolution. This balancing of increased activity with reduced specificity is advantageous with up to an average of 10 atoms of iodine per antibody molecule, after which the reduction in specificity outweighs the advantage of high activty. Using other methods for the introduction of radiolabel, it may be possible to further increase the proportion of label to antibody fragment without paying an unacceptable price in reduced immunospecificity. A further improvement may be achieved by effecting radiolabeling in the presence of the specific antigen, to insure that the antigen binding site on the antibody will be protected. The antigen is separated after labeling.

Examples of bivalent hybrid antibody fragments, e.g., $F(ab')_2$ antibody fragments obtained by chemical combination of Fab' fragments from each of two different tumor-specific antibodies are shown below. Table 1 gives an illustrative list of suitable such bivalent hybrids and tumor types for which they are advantageously used, both for detection and therapy. The first column shows the two antigens to which the two Fab' components of the hybrid are specific, each hybrid having one fragment specific to an antigenic determinant of each. In some cases, the antibody will be raised to a smaller fragment of a tumor-associated antigen to increase the specificity. For example, antibodies raised against the beta-subunit of HCG are preferred over antigens raised against HCG itself. The second column shows tumor types which preferentially concentrate each hybrid type.

TABLE 1

F(ab')$_2$ Hybrid antibody fragments and associated tumor types

| Components | Tumors |
|---|---|
| AFP/HCG | Germ cell, trophoblastic, testicular, gastrointestinal |
| CEA/CSAp | Gastrointestinal, e.g., colon, rectal, stomach |
| CEA/POA | Pancreatic, lung |
| CEA/HCG | Lung, ovarian, gastrointestinal |
| CEA/AFP | Liver, gastrointestinal, overian, testicular |
| CEA/TPA | Most |
| CEA/ferritin | Solid, hematopoietic, liver |
| CEA/calcitonin | Thyroid |
| CEA/PAP | Prostatic |
| CEA/parathormone | Parathyroid, lung |
| HCG/PBG | Trophoblastic |
| CGA/GEA | Brain |
| GFA/CMA | Brain |

Legend:
    CEA—Carcinoembryonic antigen
    HCG—Human chorionic gonadotropin (or beta-subunit)
  CSAp—Colon specific antigen-p
    POA—Pancreatic oncofetal antigen
    AFP—Alpha-fetoprotein
    TPA—Tissue Polypeptide antigen
    PAP—Prostatic acid phosphatase
    PBG—Pregnancy beta$_1$-globulin
    CGA—Common glioma antigen
    GEA—Glioembryonic antigen
    GFA—Glial fibrillary acidic protein
    CMA—Common meningioma antigen

Hybrid fragments such as those shown in Table 1 may be labeled with a single radioisotope for the detection and localization of multiple tumor types or cells, or labeled with one or more radioisotopes for therapy. Furthermore, such hybrids may be labeled with a radioisotope for detection and one or more isotopes for therapy, e.g., a radioisotope and/or a boron-containing fragment so that the localized tumor may then be irradiated with thermal neutrons in the manner disclosed below.

Elimination of the Fc fragment from an antibody to form smaller marker-specific fragments has the advantage of producing a molecule having a lower molecular weight which facilitates mobility of the fragments and their ability to pass through the blood-brain barrier. In addition, the Fc fragment is responsible for the major hyper-allergenicity of antibodies and a substantial portion of the non-specific binding. Accordingly, its cleavage reduces the danger of allergic reaction to the injection of antibodies, especially repeated injection for multiple tumor imaging or tumor radiotherapy. Since the kinetics of antibody fragments are more rapid and the binding more specific to the tumor than for whole native immunoglobulin, subtraction techniques can be dispensed with or modified for use in particular circumstances. In addition, the use of labeled antibody fragments for a more direct intra-arterial application of a labeled detection agent to the tumor supplied by the particular artery presents an important opportunity of this process, both for tumor detection and for tumor radiotherapy.

Mixtures of labeled antibody fragments may be used in a single preparation for tumor localization, detection or therapy. Fragments of different size may be used together. The mixture can use fragments specific to different antigens associated with the same tumor type to enhance localization and resolution. Alternatively, broad screening for various tumor types can be done using a mixture of fragments with diverse tumor specificities.

Analogously to the use of hybrid fragments, mixtures of labeled antibodies specific to antigens associated with the same or different tumor or tumor cell types may be used. This can enhance detection, localization and/or therapy in certain cases, and can also increase the range of a broad screen for more than one tumor or tumor cell type.

In contrast to earlier known procedures, which permit tumor localization usually only after 24 hours following administration of the radiolabeled specific antibody, the present improved subtraction technique also permits tumor detection and localization within less than 24 hours after concurrent administration of radiolabeled specific antibody and radiolabeled normal immunoglobulin. Tumor localization may be achieved as early as two hours after injection of the antibody/immunoglobulin pair, with improved resolution at 6, 12, 18 and 24 hours after administration. Because antibody fragments are diffused into the tissues much more rapidly and more rapidly localized, tumor detection and localization may be achieved within a short time of injection of the labeled fragments.

Radioactivity due to accumulation of labeled antibody fragments or their metabolites in the blood-pool or in interstitial fluids can significantly reduce the resolution of tumor localization using labeled antibody fragments specific to tumor-associated markers. In such cases, it is advantageous to inject a reference substance into the subject prior to photoscanning, the reference substance being radiolabeled with a radioisotope emitting at a different energy from the marker specific antibody fragment label and capable of independent detection by the photoscanning device. The level of activity of the reference substance is used to determine the background activity due to non-targeted specific antibody fragment, this background activity is then subtracted from the total activity of the specific antibody permitting a determination of the activity of substantially only the targeted, tumor-associated antibody.

In the reported successful noninvasive radio-immuno-detection of cancer in humans of Goldenberg et al, *N. Eng. J. Med., 298,* 1384 (1978), a subtraction technique was shown to be necessary for successful localization. However, the subtraction technique used was substantially different from that of the present invention. In the reference process, radioiodinated anti-CEA antibody was injected, and technetium-99m-labeled human serum albumin and technetium-99m-pertechnetate were injected intravenously before each imaging scan. Images were obtained with a gamma-scintillation camera and the data obtained were stored in a minicomputer.

The ratio of I-131 activity to Tc-99m-activity in non-target areas provided a standard of comparison for background non-localized antibody activity. This in turn permitted a determination of non-targeted specific antibody activity at other locations, which was then subtracted from the total specific antibody activity to yield a value for the activity of localized, targeted antibody.

The present invention includes the use of Tc-99m-labeled normal immunoglobulin G or fragments thereof and Tc-99m-labeled sulfur colloid among suitable reference substances. Preferably, however, the reference substance is the corresponding normal, indifferent immunoglobulin G (IgG) or the corresponding fragment from the same or different species as that used to prepare the specific antibody fragment used as the tumor localization agent. This normal immunoglobulin G is preferably radiolabeled with a different isotope of the same element used to label the specific antibody and is preferably injected concurrently with the radiolabeled marker-specific antibody. This has the advantage of using as a reference substance a molecular species having essentially the same kinetics of binding, distribution and metabolism as the labeled specific antibody. As a consequence, only a single injection of the reference substance is necessary, and increased resolution is achieved. The normal IgG is prepared and labeled in the same way as the specific antibody to which it corresponds.

Suitable pairs of radioisotopes, one of which may be used for labeling the specific antibody and the other of which is used to label the normal immunoglobulin include Iodine-131 and Iodine-123; Indium-111 and Indium-113m; Gallium-67 and Gallium-68; Ruthenium-97 and Ruthenium-103; or Mercury-197 and Mercury-203. Because iodine may be introduced directly by a chemical substitution reaction, and has at least five isotopes which are radioactive and detectable using a photoscanning device, iodine is preferred for radiolabeling both the specific antibody fragment and the normal immunoglobulin G reference for use in the method of the invention. Advantageously, Iodine-131 is used for labeling the specific antibody and Iodine-123 is used for labeling the normal immunoglobulin. The resultant emissions are separately detectable on two different channels of a gamma-scintillation detector.

The resultant scanning data are conveniently stored in a minicomputer and the aforementioned subtraction procedure is effected to determine the regions of excess accumulation of radiolabeled specific antibody over its ratio to labeled reference immunoglobulin in non-target areas. These values may be used to generate a related output signal, advantageously a gradation of colors on a color video screen. The photoscanning device may also include computer tomographic capabilities. This highly efficient subtraction technique with the use of highly monospecific, preferably monoclonal antibodies labeled to give the maximum balance between high activity and acceptable immunospecificity provides a tumor localization and detection method of significantly improved resolution.

Of course, the combination of highly labeled, highly specific antibody with the improved subtraction technique of this invention leads to even greater resolution. In a preferred embodiment, substantially monospecific antibody is radiolabeled with I-131 or I-123, an average of at least 2.5 and preferably from 5 to 10 atoms of iodine per antibody molecule being introduced, and the resultant highly labeled monospecific antibody is injected according to the present method. The use of monoclonal specific antibody radiolabeled with I-131 or I-123 to an average iodine content of at least 2.5 and preferably from 5 to 10 atoms per antibody molecule is also preferred.

Further improved resolution is achieved by using a radiolabeled purified normal immunoglobulin for the reference substance in the subtraction technique. Normal globulin is a mixture of globulins, some of which may bind to the specific antigen to which the radioactive antibody is directed. Therefore, it is desirable to purify the normal globulin to be used as a subtraction agent so as to remove any reactivity to the specific marker in question, and one such purification method is to adsorb the normal immunoglobulin with the specific antigen, preferably on a solid adsorbent, so that the globulins reacting with the antigen will be retained on the column and the materials passing through will be more suitable for labeling as a non-specific subtraction agent. Monoclonal non-speciifc immunoglobulin or myeloma protein itself will also have the desired purity for labeling and use as subtraction agents.

When the subtraction technique of the invention is used, the balance between high radio activity and high specificity can be struck more on the side of a somewhat lower extent of radiolabel with a correspondingly higher marker-specific immunoreactivity of the antibody. Again, the higher the marker-specific immunoreactivity, the higher the labeling can be while still preserving an advantageous balance of antibody properties. Thus, a substantially monospecific antibody having a marker-specific immunoreactivity of at least 70%, preferably at least 80%, and a cross-reactivity of less than 15%, preferably less than 10%, can be labeled with I-131 or I-123 to an extent of from 2.5, but preferably from 5 to 10 atoms of iodine per antibody molecule while still retaining a sufficient specificity after labeling to permit highly efficient localization.

The use of the present reagents in the described method, either without but preferably with the improved subtraction technique, permits either continuous, repeated or occasional monitoring of tumor locations. This has particular advantages in conjunction with the diagnosis and staging of tumors prior to surgery. In addition, the present reagents are useful during and after surgery as an indication of the extent to which complete tumor removal has been achieved. In case of metastasis, especially where there has been a proliferation of small, diffuse metastases, the high resolution of the present method permits identification of target areas for post-operative therapy. This can be effected using the therapeutic method of this invention or other known techniques, e.g., chemotherapy, radiation treatments, or multimodal therapies.

Radiolabeled marker-specific antibodies or fragments are effective for tumor therapy. After it has been determined that labeled antibodies are localized at tumor sites in a subject, a higher dose of labeled antibody, generally from 25 to 250 mCi, and preferably from 50 mCi to 150 mCi per dose is injected. Injection may be intravenous, intra-arterial, intralymphatic, intrathecal, or intracavitary, and may be repeated.

A variety of radionuclides are useful for therapy, and they may be incorporated into the specific antibody by the labeling techniques discussed above. The preferred therapeutically effective radionuclide is I-131.

Highly marker-specific antibody, preferably an antibody which is substantially monospecific, having a marker-specific immunoreactivity of at least 70%, preferably 80%, and a cross-reactivity to other antigens of less than 15%, preferably less than 10% is advantageously used in the described method. Monoclonal antibodies are preferred because of their high specificity.

Therapy using radiolabeled marker-specific antibodies or fragments is advantageously used as a primary therapeutic treatment, in combination with other therapies, e.g., radiation and chemotherapy, and as an adjunct to surgery. Where there may be small metastases which cannot be surgically removed or which may escape detection, the radiotherapeutic means of the invention provides a potent weapon capable of seeking out and destroying these tumors.

Antibody fragments will sometimes have somewhat lower immunoreactivity, and it is then advantageous to label with a lower radionuclide content, e.g., 0.1—1.0 atom per fragment molecule of, e.g., I-131. This will produce fragments having an I-131 specific activity of, 2.5—52 μCi/μg for therapy and, if necessary, for detection.

A further aspect of the present invention relates to the use of antibodies or fragments containing both a radioisotope label and an addend containing significant numbers of boron atoms, having at least the 20% natural abundance of boron-10 isotope. The boron-containing addend may be introduced by a variety of methods, preferably by coupling the antibody with a boron-rich coupling agent, such as the diazonium ion derived from 1-(4-amino-phenyl) - 1,2 - dicarbaclosododecaborane (12), according to the method of Hawthorne et al, *J. Med. Chem.*, *15*, 449 (1972). The boron-10-containing antibody is then radiolabeled according to one or more of the above precedures to produce an antibody containing both one or more radiolabels for tumor localization and/or therapy and a high content of boron-10 atoms for the absorption of thermal neutrons.

Injection of the radiolabeled and boron-labeled antibody is followed by localization of tumors using the radiolabel. The tumors are then irradiated with a well collimated beam of thermal neutrons, which are preferentially absorbed by boron-10 nuclei on the boron-containing addends, and the activated nucleus decays rapidly to lithium-7 and an alpha-particle. These resultant alpha-particles are cytotoxic, and their production in tumor cells kills the cells and causes tumor reduction.

Combination of a boron addend with one or more radiolabels on a highly marker-specific antibody provides for the first time a single agent which functions as a multimodal tumor therapeutic agent. The rapid and specific localization of these doubly labeled antibodies at the site of a tumor permits a rapid and precise definition of the areas where neutron irradiation should be focused. Moreover, as tumor cells are destroyed by the combined effects of radiation from the radiolabel and neutron-activated boron-10 emissions, and the killed tumor cells are eliminated, the progress of the radiotherapeutic treatment may be monitored by measurement of localized, radiolabeled antibody or other tumor detection methods.

The antibodies of the invention are advantageously administered in the form of injectable compositions. For general screening, and for many types of localization and therapy, injection will be intravenous, intraarterial or intrathecal. The injectable antibody solution will be administered into a vein, artery or into the spinal fluid over the course of from 2 minutes to about 1 hour, preferably from 10 minutes to 20 minutes for intraveous or intraarterial infusion. In certain cases, subcutaneous, submucosal, or intracavitary administration is advantageous. Where the tumor is supplied by a known, accessible artery, intraarterial administration is preferred for therapy. The intraarterial route can be used for longer periods of infusion of the preparation, e.g., 24 hours or longer. In addition, intrathecal administration or injection of fragments into the carotid artery may be used for tumors located in the brain. Subcutaneous, submucosal and intracavitary administration are advantageous for tumors restricted to areas close to particular regions of the skin and/or to particular body cavities.

A typical injectable composition according to the invention contains about 34 mg human serum albumin (1% USP:Parke-Davis) and from 1 to 500 micrograms of radiolabeled specific antibody per milliliter of 0.04 M phosphate buffer (pH 7.4, Bioware) containing 0.9% NaCl. Where the subtraction technique of the invention is used, a quantity of radiolabeled normal immunoglobulin roughly equal to the weight of specific antibody is also included. Other conventional pharmaceutically acceptable injection vehicles may be used where indicated, such as for intrathecal, subcutaneous, submucosal, or intracavitary injection as well as for intravenous or intraarterial injection.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following examples, all temperatures are set forth uncorrected in degrees Celsius; unless otherwise indicated, all parts and percentages are by weight.

Antibodies used in these examples are highly specific, either prepared by conventional immunization followed by complement inactivation, adsorption to remove hemagglutination components and affinity purification against cross-reactive antigens and the specific antigen, or hybridoma-derived monoclonal antibodies.

Example 1
Preparation of $^{131}$I-anti-CEA IgG(goat)

(a) CEA is obtained in high purity from hepatic metastasis of colonic carcinoma by the method of Newman et al., *Cancer Res., 34*, 2125 (1974).

(b) Purified CEA, 0.5 mg dry weight, is dissolved in 2 ml of water containing 2 mg methylated bovine serum albumin (Sigma) and the CEA solution is emulsified with an equal volume of complete Freund's adjuvant (Difco).

Equal portions of the CEA inoculum are injected subcutaneously into two separate sites on the neck of a healthy goat. Injections are administered bi-weekly until radioimmunoassay (RIA) of antiserum collected 14 days after the last injection shows an anti-CEA titer of greater than $1:10^6$.

Blood is then collected aseptically from the goat, transferred to pyrogen-free centrifuge tubes and centrifuged. The anti-CEA serum is stored at $-20°C$.

The complement of the goat anti-CEA serum is inactivated by incubation at 56°C for one hour, and freed of anti-blood group activity by repeated mixing with washed, packed type AB human RBC's with a serum/RBC ratio calculated from the hemagglutination assay, until no further hemagglutination activity can be detected. The absorbed anti-CEA serum is then dialyzed against several volumes of 0.1 M pH 7.0 phosphate buffer ($PO_4$).

(c) A colon carcinoma antigen-III (CCA-III) immunoadsorbent is prepared by conjugating the 60,000 MW fraction of the perchloric acid extracts of normal lung to cyanogen bromide-activated Sepharose 4B® (Pharmacia Fine Chemicals, Inc.), using conventional techniques. The conjugation is allowd to proceed overnight at 4°C with gentle stirring. The CCA-III-immunoadsorbent is washed with 0.05 M, pH 8.4, borate buffer and resuspended in 4 volumes of 1 M 2-aminoethanol in 0.1 M, pH 8.0 phosphate buffer. The slurry is mixed for one hour at room temperature, filtered and washed with $PO_4$.

A CCA-III immunoadsorbent column is prepared, precycled with 2 ml of 10% (v/v) normal goat serum (Gibco), and with approximately one column volume of the chaotropic agent 3 M ammonium thiocyanate, and reequilibrated with 0.1 M pH 7.0 phosphate buffer.

The CCA-III-immunoadsorbent column is inserted into an automated chromatographic system and the entire system is thoroughly washed with apyrogenic, sterile $PO_4$. The buffer reservoirs are replaced with reservoirs containing the chaotropic agent and dialysate.

The adsorbed anti-CEA serum is diluted with $PO_4$ to a volume which is 2/3 that of the void volume of the column and contains an appropriate quantity of antiserum for one cycle through the column. This volume of diluted antiserum is applied and washed into the column with sufficient $PO_4$ to give a total volume of one column void volume. The anti-serum is allowed to incubate at room temperature for 20 minutes, then the specific anti-CEA serum, unadsorbed fraction, is eluted from the column with $PO_4$. The system automatically starts the next cycle by applying a second aliquot of anti-CEA serum. The number of cycles is set to process the entire lot of antiserum.

An aliquot of the anti-CEA serum is concentrated to the original volume of the antiserum and retested by immunodiffusion. The antiserum is tested against reference CEA, a CCA-III preparation, and normal human tissue extracts and plasma. If the antiserum has a positive reaction against the CCA-III preparation, plasma or any normal human tissue extracts, it is recycled with the CCA-III immunoadsorbent column.

(d) A CEA-immunoadsorbent column is prepared by conjugating purified CEA to cyanogen bromide-activated Sepharose 4B® by a coupling procedure identical to that of the CCA-III immunoadsorbent, preparing, precycling and equilibrating a column as in part (c).

The CEA-immunoadsorbent column is introduced into the automated charomatographic system and thoroughly washed with apyrogenic $PO_4$. The quantity of anti-CEA serum to be applied with each cycle is calculated on the basis of the radioimmunoassay titration, the sample of antiserum is diluted, applied and incubated as with the CCA-III immunoadsorbent column. The serum protein including all non-reactive immunoglobulins are eluted from the column with $PO_4$ and are collected as the unadsorbed fraction.

The specific anti-CEA IgG is dissociated and eluted from the column with 3M ammonium thiocyanate in $PO_4$ and is collected as the absorbed fraction. In order to remove all traces of the ammonium thiocyanate, the adsorbed fraction is subjected to in-line dialysis by the use of hollow fiber dialysis units (Amicon or BioRad) against 1 M urea and 10% glycerol in $PO_4$. An alternate procedure for dissociation of specific antibody uses guanidine hydrochloride as a chaotropic agent and Sephadex G25® gel filtration chromatography for desalting.

The adsorbed fraction is concentrated at 4°C by ultrafiltration with PM 30 membranes (Amicon) to a volume facilitating gel filtration chromatography, e.g., a lot of 100 ml of anti-CEA serum is concentrated to approximately 20 ml. The concentrate is dialyzed against 4 changes, 4 hours each, of 100 volumes of 50 mM, pH 7.5, phosphate-buffered saline (PBS). The anti-CEA IgG preparation is sterile filtered into a sterile, pyrogen-free serum vial. An aliquot is reserved for quality control testing by RIA and immunodiffusion.

(e) A Sephacryl S-200® (Pharmacia) column is prepared by washing the gel 5 times with sterile, non-pyrogenic 50 mM, pH 7.5, PBS. The columns are dry-heat sterilized, 180°C for 3 hours. The column sizes used are 2.6×90 cm or 5.0×90 cm depending on the lot size of the antibody. The prepared column is placed in a refrigerated unit or cold room, equilibrated to 4°C and thoroughly washed with sterile 50 mM PBS. The column is attached to a U.V. monitor and calibrated with commercial normal goat IgG (Pentex), 50 mg in 20 ml for a 5×90 cm column and 20 mg in 5 ml for the 2.6×90 cm column. It is then washed with three column volumes of PBS.

The lot of goat anti-CEA IgG is applied to the column and eluted with PBS at a flow rate of 6 ml/cm²/hr. The fractions containing IgG protein are pooled and concentrated to approximately 5 mg IgG protein/ml, $E_{1\,cm}^{1\%}=14$ at 280 mμ, dialyzed against PBS, sterile filtered with 0.2 micron Millex® units (Millipore) and stored refridgerated in one ml aliquots, approximately 5 mg IgG protein/ml, with sodium azide as a biostatic agent.

(f) Goat anti-CEA IgG, 7.2—16.8 μg IgG per mCi $^{131}$I, is injected into a radionuclide vial containing $^{131}$I (Amersham-Searle).

Chloramine-T and sodium metabisulfite solutions are prepared by the injection of 5 ml of sterile pyrogen-free 0.04 M pH 7.4 phosphate buffer into each of two vials containing 10 mg of chloramine-T and 50 mg of bisulfite, respectively. Chloramine-T solution is injected, 10 μg/mCi $^{131}$I, into the radionuclide vial. Sodium metabisulfite solution, 5 times the amount of chloramine-T, is injected into the vial exactly 90 seconds after the chloramine-T. The mixture is removed from the reaction vial with a sterile syringe, the reaction vial is rinsed twice with 1% normal human serum albumin, and the rinses combined with the reaction mixture.

The sample of $^{131}$I-anti-CEA IgG is applied to a PD-10 Sephadex G-25® column which is pre-equilibrated with 1% normal human serum albumin in PBS, eluted with approximately 4.5 ml of 1% normal human serum albumin in PBS, monitored with a shielded gamma detector (Eberline), collected and diluted to a predetermined concentration for storage and use.

The resultant $^{131}$I-anti-CEA IgG has an average of from 3 to 7 atoms of iodine per antibody molecule. Random aliquots from each batch are separately tested for sterility, pyrogenicity, toxicity and other quality control variables.

Example 2
Preparation of monoclonal $^{131}$I-anti-CEA IgG

Female, 6-month-old, Balb/C mice are injected with 10—100 µg carcinoembryonic antigen intraperitoneally, whereby the CEA is mixed in an equal volume (10—100 µl) of incomplete Freund's adjuvant. This is repeated one week later, and again two weeks later, but lastly using the intravenous route without adjuvent. Three to four days later, the mice are killed by cervical dislocation. The optimum time for obtaining antibody against a given antigen varies with the antigen, the route of administration, and the timing of immunization, as well as the interval between the last booster injection and the removal of the spleen cells.

The spleens are removed and placed in 60 mm Petri dishes containing either serum-free medium or Dulbecco's Modified Eagle's Medium (DMEM) with 20% fetal calf serum, at room temperature, and minced with scissors to disperse the cells. The cells are further liberated by agitation for 1—2 min on a Vortex mixer. The spleen cells are removed to a conical centrifuge tube and pelleted at 1,000 rpm in an IEC-MS2 centrifuge, the supernant is removed, the pellet tapped loose, and then resuspended in 5 ml of cold 0.17 $NH_4Cl$ for 10 min to lyse red blood cells. Chilled DMEM with 20% fetal calf serum is added and the cells pelleted, and then again suspended in 10 ml DMEM supplemented with 20% fetal calf serum.

The myeloma cell lines used for fusion are maintained in stationary suspension cultures in DMEM with high glucose (4.5 g/L) and 20% fetal calf serum, in 5—10% $CO_2$ at a cell concentration between 100,000 and 1,000,000 per ml. The myeloma (plasmacytoma) cell lines can be P3-X63-Ag8, which is a Balb/C plasmacytoma derived from MOPC-21, reported by Svasti and Milstein, *Biochem. J., 128*, 427—444 (1972), or a derivative thereof known as FO (Fazekas de St. Groth and Scheidegger, Basle Institute of Immunology, Basle, Switzerland), or 45.6TG1.7, which is a Balb/C line derived from MPC-11, reported by Margulies et al., *Cell, 8*, 405—415 (1976). All of these lines lack the enzyme hypoxanthine phosphoribosyl transferase (HPRT; E.C. 2.4.2.8) and are thus killed in a selective medium containing hypoxanthine, aminopterin, and thymidine (HAT), as described by Littlefield, *Science, 145*, 709—710 (1964).

The spleen cells obtained from the immunized animal are then fused with the plasmacytoma cells by using polyethylene glycol according to an adaptation of the method of Gelfer et al., *Somatic Cell Genetic., 3*, 231—236 (1977). For example, a 30% polyethylene glycol solution is made by heating sterile polyethylene glycol 4000 (Merck, molecular weight of about 4,000) (0.5 g Polyethylene glycol+0.05 ml dimethylsulfoxide (DMSO)+0.5 ml distilled water) and DMEM without serum to 41°C and mixing 3 ml of polyethylene glycol with 7 ml DMEM without serum, pH 7.4—7.6, and kept at 37°C until use. Fusions are made at room temperature. The myeloma cells ($10^6$—$10^7$) are washed twice in serum-free medium and then mixed with 1—$3\times10^7$—1—$3\times10^8$ spleen cells in 50 ml conical bottom centrifuge tubes (Falcon 2070). The cells are centrifuged at 250×g for 5 min, and the supernantant fluid is carefully aspirated. An amount of 0.2 ml of the polyethylene glycol preparation is added, and the tube is gently agitated by hand to resuspend the cells. Next, the cells are centrifuged for 3 min at 250×g and again at 400×g for another 3 min, and then kept undisturbed for an additional 3 min. The cells are exposed to polyethylene glycol for about 8 minutes. Thereafter, about 5 ml of serum-free medium is added to the tube, the cells are resuspended gently, and then repelleted by centrifugation at 250×g for 5 min. The supernatant is removed and the cells are suspended in 20 ml of serum-containing medium and incubated at 37°C in a humidified incubator for 48 hr. before being placed in microplates to which HAT medium is added. Alternatively, the cells are immediately suspended in 30 ml of a medium consisting of DMEM, 10% NCTC 109 medium (Microbiological Associates), 20% fetal calf serum (Gibco), 0.2 units bovine insulin/ml (Sigma), 0.45 mM pyruvate, 1 mM oxaloacetate, and antibiotics of choice. Thymidine ($1.6\times10^{-5}$ M) and hypoxanthine ($1\times10^{-4}$ M) are added. The cells in this medium are distributed into 6 microplates (Linbro FB 96 TC) with 1 drop (about 50 µl) per well. The next day 1 drop of the above-specified medium containing thymidine and hypoxanthine, now with aminopterin ($8\times10^{-7}$ M), is added to each well. Two drops of the medium of above is added 6—7 days later and clones appear microscopically between 10 and 20 days. The hypoxanthine-aminopterin-thymidine (HAT) medium can also be added immediately after the fusion, or at a later time. An improvement in the number of hybrids obtained is made when a feeder layer is added to each microwell. Here, human fetal fibroblasts are irradiated with 4500 r, and 1,000—2,000 such cells are added to each well, either the day before the fusion or directly to the fused cells and so dispensed with them into the microwells. After clones have appeared macroscopically, the medium is changed by removing most of the medium and adding fresh medium. After a second change of medium, the medium is left there for at least 4 days and then collected for assays of antibody activity and specificity by conventional assays.

Large amounts of antibody are obtained from spent culture medium harvested from 150 mm plates or roller bottles. The medium is subsequently concentrated by means of a hollow-fiber concentrator (Amicon). Also, antibody is obtained from the ascites fluid of athymic (nude) mice (nu/nu) that were injected 2—3 weeks previously with about 1 billion cloned hybridoma cells. The ascites fluid is diluted with saline by flushing the peritoneal cavity of each mouse with saline, and the diluted fluids from each mouse are pooled.

The monoclonal anti-CEA IgG is radiolabeled with I-131 as in Example 1(f).

Example 3
Preparation of $^{123}$I-IgG(goat)

Normal goat immunoglobulin G (IgG) (Miles) is affinity purified against cyanogen bromide-linked CEA and labeled with I-123, using the procedure of Example 1(f), except that I-123 is substituted for I-131, with proportional changes in the reagents to account for differences in specific activity. Carrier iodine may be added to adjust the specific activity of the labeled antibody to, e.g., 100—500 µCi/µg. The amount of added carrier iodine will determine the specific activity of the labeled antibody molecules, for a given number of iodine atoms per antibody.

Example 4
Preparation of $^{131}$I-anti-CEA-$^{10}$B IgG

(a) Anti-CEA IgG prepared according to Examples 1 or 2 is reacted with a 20-fold molar excess of the diszonium salt of 1-(3-amino-phenyl)-1,2-dicarbacloso - dodecarborane (12) having a natural abundance of Boron-10 isotope (20%), using the procedure of Hawthorne et al., *J. Med. Chem.*, *15*, 449 (1972). The resultant antibody has an average of from 2 to 10 diazo-linked carborane residues or from 4 to 20 Boron-10 atoms per antibody molecule.

(b) The anti-CEA-$^{10}$B IgG of part (a) is radiolabeled with I-131 as in Example 1(f), to introduce an average of from 1 to 10 atoms of iodine per antibody molecule.

Example 5
Preparation of injectable compositions

Sterile, pyrogen-free solutions are prepared as shown.

(a) A sterile solution containing, per ml:
(1) 34 mg Human Serum Albumin (HSA) (1%, USP, Parke-Davis)
(2) 0.04 M phosphate buffer, pH 7.4 (Bioware)
(3) 0.9% NaCl
(4) 10 µg $^{131}$I-anti-CEA IgG (goat) prepared according to Example 1 (average of about 5 atoms of Iodine/molecule, specific activity of about 80 µCi/µg).

The labeled antibody of Example 1 is stored in a solution of (1), (2) and (3) at a concentration of 20 µg/ml and diluted with an equal volume of 1% HSA is phosphate buffered saline (PBS) to prepare this solution.

(b) A sterile solution according to the precedure of part (a) except that it further contains 15.1 µg/ml of $^{123}$I-IgG as prepared in Example 3. The $^{123}$I-IgG, at a specific activity of 500 µCi/µg, is stored in phosphate buffered saline containing 1% HSA at a concentration of 10.2 µg/ml. An equal volume of this solution is used in place of 1% HSA in PBS in the procedure of part (a).

(c) A sterile solution according to the precedure of part (b) except that the antibody is the $^{131}$I-anti-CEA IgG (monoclonal) prepared according to Example 2, stored in 1% HSA in PBS at a concentration of 20 µg/ml and having comparable activity.

(d) A sterile solution according to the procedure of part (b) except that the antibody is the $^{131}$I-anti-CEA-$^{10}$B IgG prepared according to Example 4, having an average of 5 diazo-linked carborane residues and 1 atom of iodine per antibody molecule, and a specific activity of about 16 µCi/µg. The final solution contains 50.4 µg/ml of the antibody.

Example 6
Tumor localization

Radioiodinated anti-CEA IgG is administered to patients with suspected tumors. The patient is pretested for anaphylactic hypersensitivity to goat IgG or myeloma IgG. To block thyroid uptake of I-131 or I-123, Lugol's solution (Purepack) is administered by mouth, 5 drops twice daily for seven days beginning one day before injection of the radioactively labeled antibody.

Localization is effected according to the procedure of Goldenberg et al., *N. Eng. J. Med.*, *298*, 1384 (1978), by infusion of 3.5 ml of a solution of $^{131}$I-anti-CEA IgG containing $^{123}$I-IgG prepared according to Example 5(b) or 5(c) in 20 ml of sterile physiological saline over a period of from 10 minutes to 45 minutes. No Tc-99m compounds are used, the subtraction technique being adapted in a conventional fashion to discriminate between $^{131}$I and $^{123}$I. Scans are taken immediately and at 2, 8, 12, 24, 48 and 72 hours after injection of the antibody is completed.

Significant localization is seen after 2 hours, with improved resolution with time, tending to plateau between 8 and 24 hours after injection. No additional background $^{123}$I-IgG is added. The CEA-selectivity of this method is comparable to the earlier Goldenberg et al, method, but the resolution, rapidity and convenience are enhanced significantly.

Example 7
Tumor therapy

(a) A patient having an ovarian cancer, optionally detected and localized by the procedure of Example 6, is injected by intravenous infusion with 150 mCi of the solution of Example 5(a) in 50 ml of sterile physiological saline. Reduction in tumor size is observed within 20 days. The dose is repeated at intervals adjusted on an individual basis.

(b) A patient having a cervical cancer optionally detected and localized by the procedure of Example 6 is injected with an amount of the solution of Example 5(d) (in 50 ml of sterile physiological saline) sufficient to provide 2.8 mCi of $^{131}$I activity based on a 70 kg patient weight.

The tumor is precisely localized 12 hours after injection using the procedure of Example 6. A well collimated beam of thermal neutrons is focused on the defined tumor locations. Irradiation with $3\times10^{12}$ neutrons per cm$^2$ is effected for each tumor locus, and is optionally repeated with administration of the tumor-localizing antibody, with or without the radiolabel, at intervals adjusted on an individual basis. Successful tumor reduction is observed.

Example 8
Preparation of $^{131}$I-anti-HCG IgG (goat)

- (a) Antibodies to HCG (anti-HCG) are prepared by the method of Bagshawe, in "Methods in Investigative and Diagnostic Endocrinology, Part III, Peptide Hormones", Bearson et al, Eds., pages 756—763 (North-Holland, Amsterdam, 1973).

Antibodies to the β-subunit of HCG (anti-HCG-β IgG) are prepared by the method of Vaitukaitis et al, *Am. J. Obstet, Gynecol., 113*, 751 (1972).

(b) The antibodies prepared in part (a) are further purified as serum solutions. The procedure is the same for anti-HCG IgG and anti-HCG-β IgG.

The complement of anti-HCG (or anti-HCG-β) serum is inactivated by incubation at 56°C for one hour, and freed of anti-blood AB human RBC's with a serum/RBC ratio calculated from the hemagglutination assay, until no further hemagglutination activity can be detected. The adsorbed anti-HCG serum is then dialyzed against several volumes of 0.1 M pH 7.0 phosphate buffer (PO$_4$).

(c) A human urinary protein (HUP) immunoadsorbent is prepared by conjugating purified HUP to cyanogen bromide-activated Sepharose 4B, an HUP-immunoadsorbent column is prepared, and the anti-HCG (or anti-HCG-β) is purified and tested analogously to the procedure of Example 1(c).

(d) An HCG-immunoadsorbent column is prepared by conjugating purified HCG to cyanogen bromide-activated Sepharose 4B by a coupling procedure identical to that of the HUP immunoadsorbent, preparing, precycling and equilibrating a column as in part (c).

The HCG-immunoadsorbent column is introduced into the automated chromatographic system and anti-HCG serum from part (c) above is purified analogously to the procedure of Example 1(d).

The anti-HCG IgG preparation is sterile filtered into a sterile, pyrogen-free serum vial. An aliquot is reserved for quality control testing by RIA and immunodiffusion.

(e) A Sephacryl S-200 (Pharmacia) column is prepared, and the lot of goat anti-HCG IgG from part (d) above is purified analogously to the procedure of Example 1(e), and stored refrigerated in one ml aliquots, approximately 5 mg IgG protein/ml, with sodium azide as a biostatic agent.

(f) Goat anti-HCG IgG, 7.2—16.8 µg IgG per mCi $^{131}$I, is injected into a radionuclide vial containing $^{131}$I (Amersham-Searle), labeled, purified and stored analogously to the procedure of Example 1(f).

The resultant $^{131}$I-anti-HCG IgG or $^{131}$I-anti-HCG-β IgG has an average of from 3 to 7 atoms of iodine per antibody molecule. Random aliquots from each batch are separately tested for sterility, pyrogenicity, toxicity and other quality control variables.

Example 9
(a) Preparation of $^{131}$I-anti-AFP IgG (goat) Hyperimmune goat antiserum is prepared by repeated intradermal injections of human AFP purified by the procedure of Nishi, *cancer Res., 30*, 2507 (1970). The specificity of the antiserum is confirmed by double gel diffusion, immuno-electrophoresis and radioimmunoassay. Immunoelectrophoretically pure IgG is prepared by DEAE-cellulose chromatography of 10 ml antiserum, followed by concentration over an Amicon PM-30 membrane. At a concentration of 4.2 mg/ml, the goat anti-AFP IgG has a radio-immunoassay titer of $3 \times 10^5$ using 50 percent binding of a 2 ng labeled antigen as the end point.

The antibody is radiolabeled with I-131 analogously to the procedure of Example 1(f), purified and stored as described in that Example.

(b) Preparation of $^{131}$I-anti-CSAp IgG (goat)

Anti-CSAp serum is prepared by homogenizing GW-39 human colon cancer xenograft tumors in 5 volumes of deionized water (w/v) under ice water by a polytron at full speed for 2 minutes. The homogenate is then centrifuged at 48,000×g for 1 hour. The clear supernatant is used as immunogen. The protein content of the immunogen is 7 mg/ml as estimated by Lowry's method. A goat is immunized with 5 ml (35 mg protein) of the homogenate emulsified in 5 ml of complete Freund's adjuvant, given subcutaneously in the neck of the goat. Booster immunizations are continued at 3—4 weeks intervals. The goat is trial bled each month and the serum is tested for antibody production. Bleeding of the 10th month and of the later dates are processed by affinity methods for preparation of specific anti-CSAp antibodies.

The anti-CSAp antibodies are affinity purified against various organ and tissue extracts. Tissue extracts of human colon cancer, human spleen and lung and hamster liver and kidney are made in 5 volumes of deionized water (w/v) under ice water by a polytron at full speed for 2 minutes. The homogenates are centrifuged at 48,000×g for 1 hour. The clear supernatants are collected, lyophilized and redissolved in water to obtain proper protein concentration for conjugation of the antigens to Sepharose 4B by the cyanogen bromide method. Similarly, human and hamster sera are adjusted for protein prior to conjugation to Sepharose 4B. GW-39 water homogenate and its 48,000 g supernatant are treated with 90% liquid phenol for preparation of CSA's, as reported in Goldenberg et al, *Cancer Res., 36*, 3455 (1976), and conjugated to Sepharose 4B.

Affinity adsorptions are preformed analogously to the procedure of Example 1(c)—(e). Crude goat anti-CSAp (4 ml) is applied to a column containing 100 ml of Sepharose 4B conjugated with colon cancer extract and allowed to flow downwards. The flow is stopped when protein appears in the effluent and then the antiserum is allowed to react for 30 minutes with the immunoadsorbent and then the gel is washed with 0.1M PO₄, pH 7.0 buffer. The adsorbed immunoglobulins are eluted with 3M NH₄SCN in 0.1M PO₄ pH 7.0 buffer. The eluted antibodies are immediately dialysed on Amicon PM-30 membrane and equilibrated with 0.1 M PO₄, pH 7.0 buffer followed by 1.0M urea and 10% glycerol in 0.05M PO₄, pH 7.0, and finally with 0.1M PO₄, pH 7.0 buffer. The antibodies are then passed through a mixture of 75 ml Sepharose 4B conjugated with human spleen, lung and serum. After allowing the antibodies to react with the immunoadsorbent, the column was eluted with 0.1M PO₄, pH 7.0 buffer. The antibodies are concentrated on an Amicon PM-30 membrane and passed through a 75 ml immunoadsorbent column containing a mixture of hamster liver, kidney and serum antigens and processed in the above manner.

Finally, the antibodies are applied to a 320 ml immunoadsorbent column made with a phenol extract of GW-39 tumor. After incubating the antibodies for 30 minutes the column is eluted with 0.1M PO₄, pH 7.0 buffer. The antibodies are then concentrated and equilibrated with 0.05M PO₄, pH 7.5 buffer on an Amicon PM-30 membrane. Affinity purified anti-CSAp antibodies obtained thus are applied to a Sephadex G-200 (2.5×100 cm) column and fractionated using 0.05M, pH 7.5 buffer. The IgG fraction is pooled and concentrated on Amicon PM-30. The protein of anti-CSAp IgG is adjusted to 2.8 mg/ml, and stored at −20°C.

The radioiodination of anti-CSAp with I-131 is effected analogously to the procedure of Example 1(f), and the ¹³¹I-anti-CSAp IgG is purified and stored as in that Example.

(c) Using analogous procedures to those of Examples 1, 8 and this Example, except for the replacement of CEA, HCG, AFP or CSAp by one of prostatic acid phosphatase, pancreatic oncofetal antigen, placental alkaline phosphatase, pregnancy beta₁-globulin, parathormone, calcitonin, tissue polypeptide antigen, T-antigen, beta₂-microglobulin, galactosyl transferase-II (GT-II), gp-52 viral-associated antigen, ovarian cystadenocarcinoma-associated antigen (OCAA), overian tumor-specific antigen (OCA), cervical cancer antigens (CA-58, CCA, TA-4), basic fetoprotein (BFP), terminal deoxynucleotidyl transferase (TdT), cytoplasmic melanoma-associated antigens, human astrocytoma-associated antigen (HAAA), common glioma antigen (CGA), glioembryonic antigen (GEA), glial fibrillary acidic protein (GFA), common meningioma antigen (CMA) and tumor angiogenesis factor (TAF), and with appropriate affinity purification, labeled antibodies to these antigens are obtained.

Example 10
Preparation of monoclonal ¹³³I-anti-AFP IgG

Monoclonal ¹³³I-anti-AFP IgG is prepared analogously to the procedure of Example 2, except that AFP antigen is used instead of CEA.

The monoclonal anti-AFP IgG is radiolabeled with I-133 analogously to the procedure of Example 1(f).

Example 11
Preparation of ¹²³I-IgG (goat)

Normal goat IgG (Miles) is affinity purified against cyanogen bromide-linked HCG, AFP and CSAp and labeled with I-123 analogously to the procedure of Example 1(f), except that I-123 is substituted for I-131, with proportional changes in the reagents to account for differences in specific activity, as in Example 3.

Example 12
Preparation of ¹³¹I-anti-AFP-¹⁰B IgG

(a) Anti-AFP IgG prepared according to Example 9(a) is reacted with a 20-fold molar excess of the diszonium salt of 1 - (4 - aminophenyl) - 1,2 - dicarbacloso - dodecarborane (12) having a natural abundance of Boron-10 isotope (20%), analogously to the procedure of Example 4(a). The resultant antibody has an average of from 2 to 10 diazolinked carborane residues or from 4 to 20 Boron-10 atoms per antibody molecule.

(b) The anti-AFP-¹⁰B of part (a) of radiolabeled with I-131 analogously to the procedure of Example 1(f), to introduce an average of from 1 to 10 atoms of iodine per antibody molecule.

Example 13
Preparation of injectable compositions

Sterile, pyrogen-free solutions are prepared as shown.

(a) A sterile solution containing, per ml:
(1) 34 mg Human Serum Albumin (HSA) (1%, USP, Parke-Davis)
(2) 0.04M phosphate buffer, pH 7.4 (Bioware)
(3) 0.9% NaCl
(4) 10 μg ¹³¹I-anti-HCG IgG (goat) prepared according to Example 8 (average of about 5 atoms of iodine/molecule, specific activity of about 80 μCi/μg).

The labeled antibody of Example 8 is stored in a solution of (1), (2) and (3) at a concentration of 20 μg/ml and diluted with an equal volume of 1% HSA in phosphate buffered saline (PBS) to prepare this solution.

(b) A sterile solution according to the procedure of part (a) except that it further contains 5.1 μg/ml of ¹²³I-IgG as prepared in Example 11. The ¹²³I-IgG, at a specific activity of 500 μCi/μg, is stored in phosphate buffered saline containing 1% HSA at a concentration of 10.2 μg/ml. An equal volume of this solution is used in place of 1% HSA in PBS in the procedure of part (a).

(c) A sterile solution according to the procedure of part (b) except that the antibody is the ¹³¹I-anti-AFP IgG (goat) prepared according to Example 9(a), stored in 1% HSA in PBS at a concentration of 20 μg/ml and having comparable activity.

(d) A sterile solution according to the procedure of part (b) except that it further contains 10 μg/ml of ¹³³I-anti-AFP IgG (monoclonal) prepared according to Example 10. The [131]I-anti-HCG IgG, [123]I-IgG and [133]I-anti-AFP IgG are each stored at a concentration 3 times that of the desired specific activity in the final solution, and an equal volume of each is used to make the sterile solution.

(e) A sterile solution according to the procedure of part (b) except that the antibody is the [131]I-anti-AFP-$^{10}$B IgG prepared according to Example 12, having an average of 5 diazo-linked carborane groups and an average of 1 iodine atom per antibody molecule, and a specific activity of 16 µCi/µg. The solution contains 50.4 µg/ml of the antibody.

Example 14
Tumor localization
Radioiodinated antibody is administered to patients with suspected tumors. The patient is pre-tested for anaphylactic hypersensitivity to goat IgG. To block thyroid uptake of I-131 or I-123, Lugol's solution (Purepack) is administered by mouth, 5 drops twice daily for seven days beginning one day before injection of the radioactively labeled antibody.

(a) Localization is effected according to the procedure of Goldenberg et al., *N. Eng. J. Med.,* *298*, 1384 (1978), by infusion of 3.5 ml of a solution of [131]I-anti-HCG IgG containing [123]I-IgG prepared according to Example 13(b) in 20 ml of sterile physiological saline over a period of from 10 minutes to 20 minutes. No Tc-99m compounds are used, the subtraction technique being adapted in a conventional fashion to discriminate between I-131 and I-123. Scans are taken immediately and at 2, 8, 12, 24, 48 and 72 hours after injection of the antibody is completed.

Data analysis involves storing the photoscanning data in a computer, equalizing the activity level of the labeled normal immunoglobulin with that of the labeled antibody in at least one target area and calculating a background level value for the labeled antibody for each data point; subtracting the resultant background value from the total antibody activity, pixel-by-pixel, to generate a value for the activity of targeted antibody for each data point; and using the resultant generated values for targeted antibody activity to generate a related output signal.

Significant localization is seen after 2 hours, with resolution improving with time, tending to plateau between 8 and 24 hours after injection. No additional background [123]I-IgG is added. The HCG-selectivity of this method is comparable to the CEA-selectivity of the earlier Goldenberg et al method, but the resolution, rapidity and convenience are enhanced significantly.

Imaging is particularly successful in patients with HCG-secreting germ cell tumors of the testes. Secondary pelvic and abdominal tumors in these patients are also successfully located, some of which, e.g., abdominal metastasis, are difficult to impossible to detect by abdominal computerized tomography, intravenous pyelography and inferior venacavography.

(b) The procedure of part (a) is followed, except that the antibody is [131]I-anti-AFP IgG in a solution containing [123]I-IgG as prepared in Example 13(c).

Imaging is comparable to that in part (a), being especially successful in patients with testicular and hepatic cancers. Secondary lung and abdominal metastases are well localized despite serum AFP levels which are often highly elevated.

(c) The procedure of part (a) is followed except that the antibody solution is 3.5 ml of the [131]I-anti-HCG IgG, [133]I-anti-AFP IgG and [123]I-IgG prepared according to Example 13(d). The photoscan data are analyzed by conventional means, separating the I-131, I-133 and I-123 radiation, storing and using the equalized I-123/I-131 and I-123/I-133 ratios to determine the background values for non-targeted antibodies, these being subtracted to compute targeted activities for each antibody separately.

Imaging is successful for all tumor types successfully imaged with the separate antibody scans of parts (a) and (b). The combination scan gives enhanced localization and resolution in many cases, especially embryonal cancers.

Example 15
Tumor therapy
(a) A patient having germ-cell cancer of the testis, optionally detected and localized by the procedure of Example 14(a), is injected by intravenous infusion with 150 mCi of the solution of Example 13(a) in 50 ml of sterile physiological saline. Reduction in tumor size is observed within 20 days. The dose is repeated at intervals adjusted on an individual basis.

(b) A patient having a hepatic cancer optionally detected and localized by the procedure of Example 14(b), is injected with an amount of the solution of Example 13(e) (in 50 ml of sterile physiological saline) sufficient to provide 2.8 mCi of [131]I activity based on a 70 kg patient weight.

The tumor is precisely localized 12 hours after injection using the procedure of Example 13. A well collimated beam of thermal neutrons is focused on the defined tumor locations. Irradiation with $3 \times 10^{12}$ neutrons per cm$^2$ is effected for each tumor locus, and is optionally repeated with administration of the tumor-localizing antibody, with or without the radiolabel, at intervals adjusted on an individual basis. Successful tumor reduction is observed.

Example 16
Preparation and labeling of antibody F(ab')$_2$ and Fab' fragments
(a) Affinity purified goat anti-CEA IgG as prepared in Example 1, 2 ml of 15 mg/ml in phosphate buffered saline, is dialyzed against 1 liter of sodium acetate buffer, 100 mM, pH 4.0 (NaAc) for 24 hours at 4°C. The antibody solution is transferred to a screw top tube, preheated to 37°C and incubated with hog pepsin (Sigma), 0.1 ml of 3 mg/ml in NaAc, for 16 hours at 37°C. The digest, including any precipitate, is dialyzed against sodium phosphate buffer, 100 mM, pH 7.5 (PO$_4$)

for 6 hours at 4°C. The resultant solution is chromatographed on Sephadex G-100 with $PO_4$, the $F(ab')_2$ fragments eluting in the second protein peak. The $F(ab')_2$ fractions are combined, concentrated by ultrafiltration and dialyzed against $PO_4$, 50 mM, pH 7.5, and stored at −20°C.

(b) The resultant solution of part (a) prior to gel filtration chromatography is treated with 2-mercaptoethanol, 7 µl per ml of sample, and incubated for 0.5 hour at 4°C with constant stirring. Iodoacetamide, 1M, 0.135 ml per ml of sample, is added and the mixture incubated for 1 hour at 4°C with stirring, followed by clarification by centrifugation.

The resultant solution of crude Fab' fragments is purified by gel filtration on Sephadex G-100, the anti-CEA Fab' fragments eluting in the second protein peak. The fractions of peak two are pooled, concentrated by ultrafiltration, dialyzed against $PO_4$, 50 mM, pH 7.5, and stored at −20°C.

(c) By the procedure of part (a), $F(ab')_2$ fragments of anti-HCG, anti-AFP, anti-CSAp, anti-CGA, anti-GEA, anti-GFA, anti-CMA and anti-HAAA are prepared.

(d) By the procedures of parts (a) and (b), the Fab' fragments of anti-HCG, anti-AFP, anti-CSAp, anti-CGA, anti-GEA, anti-GFA, anti-CMA and anti-HAAA are prepared.

(e) 4.4—10.2 µg of a $F(ab')_2$ fragment from parts a or c above, or 2.4—5.6 µg of a Fab' fragment from parts b or d above, per mCi $^{131}I$, is injected into a radionuclide vial containing $^{131}I$ (Amersham-Searle), and radiolabeled analogously to the procedure of Example 1(f).

The resultant $^{131}I$-antibody $F(ab')_2$ has an average of from 3 to 7 atoms of iodine per fragment. Random aliquots from each batch are separately tested for sterility, pyrogenicity, toxicity and other quality control variables.

Example 17
Preparation and labeling of hybrid fragments

(a) A mixture of equal quantities of anti-CEA and anti-CSAp $F(ab')_2$ fragments prepared in Example 16(c), in the form of 15 mg/ml solutions in pH 5.0 NaAc buffer is treated with a solution of 0.01 M 2-mercaptoethylamine hydrochloride, 7 µl per ml of sample and incubated for 1 hour at 37°C with constant stirring. The mixture is passed through an ion exchange column (IR 120) at pH 5 and 40°C to remove the reducing agent, the resultant solution adjusted to pH 8 and stirred for 2 hours with gentle stirring and passage of oxgyen through the solution. The solution is then dialyzed against $PO_4$ for 6 hours at 4°C and chromatographed on Sephadex G-100 with $PO_4$ as in the final step of Example 16(a).

The fraction containing $F(ab')_2$ hybrids elutes in the second protein peak. This fraction is subjected to affinity chromatography on separate Sepharose 4B columns with cyanogen bromide linked CEA and CSAp. That fraction retained by both columns is chaotropically dissociated, re-chromatographed on Sephadex G-100 and further purified by precipitation with sodium sulfate, dissolution and dialysis against $PO_4$, and the solution of anti-CEA/CSAp $F(ab')_2$ is stored at −20°C.

(b) By the procedure of part (a), the following hybrid $F(ab')_2$ fragments are prepared, the component Fab' arms being indicated by their antibody source:
(i) anti-AFP/HCG $F(ab')_2$
(ii) anti-CEA/PAP $F(ab')_2$
(iii) anti-CGA/GEA $F(ab')_2$

(c) The fragments prepared in parts (a) and (b) are each radiolabeled with either I-131 or I-133 analogously to the procedure of Example 1(f).

Example 18
Preparation and labeling of normal IgG fragments

Normal goat IgG (Miles) is affinity purified against cyanogen bromide-linked HCG, AFP, CSAp, CEA, PAP, GEA and CGA, fragmented by the procedures of Example 16, and the fragments radiolabeled with I-123 analogously to the procedure of Example 1(f), except that I-123 is substituted for I-131 with proportional changes in the reagents to account for difference in specific activity, as in Example 3.

Example 19
Preparation and labeling of Boron-10-containing antibody fragments

(a) An antibody fragment, e.g., anti-CEA $F(ab')_2$ prepared according to Example 16, is reacted with a 20-fold molar excess of the diazonium salt of 1-(3 - aminophenyl) - 1,2 - dicarba - closo - dodecaborane (12) having a natural abundance of Boron-10 isotrope (20%), analogously to the procedure of Example 4(a). The resultant fragment has an average of from 2 to 10 diazo-linked carborane residues or from 4 to 20 Boron-10 atoms per antibody fragment.

(b) The anti-CEA-$^{10}B$-$F(ab')_2$ of part (a) is radiolabeled with I-131 analogously to the procedure of Example (f), to introduce an average of from 1 to 10 atoms of iodine per antibody fragment.

(c) The $F(ab')_2$ fragments of Example 16(c) are each reacted to add a carborane addend according to the procedure of part (a) of this example, and the resultant Boron-10-containing fragments are radioiodinated analogously to the procedure of Example 1(f), to achieve labeling of an average of 1—10 atoms of iodine per antibody fragment.

(d) The procedure of Example 16(b) is followed, except that the N-iodoacetamide of 1 - (4 - aminophenyl) - 1,2 - dicarba - closo - dodecarborane (12) is used in place of iodoacetamide. The amide is prepared by reacting the amine with iodoacetyl chloride in the presence of base, according to conventional procedures. The resultant Fab' fragments bear two carborano-phenyl-substituted acetamide groups on the sulfhydryl groups liberated in the reductive cleavage. The Boron-10-containing fragments are radioiodinated analogously to the procedure of Example 1(f), to achieve labeling of an average of 1—10 atoms of iodine per antibody fragment.

(e) The hybrid $F(ab')_2$ fragments prepared according to Examples 17(a) and 17(b) are reacted as in part (a) of this example and then radioiodinated analogously to the procedure of Example 1(f), to achieve labeling of an average of 1—10 atoms of iodine per antibody fragment.

Example 20
Preparation of injectable compositions
Sterile, pyrogen-free solutions are prepared as shown.

(a) a sterile solution containing, per ml:

(1) 34 mg Human Serum Albumin (HSA) (1%, USP, Parke-Davis)

(2) 0.04 M phosphate buffer, pH 7.4 (Bioware)

(3) 0.9% NaCl

(4) 6.1 μg $^{131}$I-anti-CEA $F(ab')_2$ (goat) prepared according to Example 16 (average of about 5 atoms of iodine/molecule, specific activity of about 125 μCi/μg).

The labeled antibody of Example 16 is stored in a solution of (1), (2) and (3) at a concentration of 24.4 μg/ml and diluted with three volumes of 1% HSA in phosphate buffered saline (PBS) to prepare this solution.

(b) A sterile solution according to the procedure of part (a) except that it further contains 5.2 μg/ml of $^{123}$I-$F(ab')_2$, at a specific activity of 500 μCi/μg, as prepared in Example 18. The $^{123}$I-$F(ab')_2$ is stored in phosphate-buffered saline containing 1% HSA at a concentration of 20.8 μg/ml. An equal volume of this solution is used in place of one volume of 1% HSA in PBS in the procedure of part (a).

(c) A sterile solution according to the procedure of part (b) except that the antibody is 10.1 μg/ml of the $^{131}$I-anti AFP Fab' prepared according to Example 16, stored in 1% HSA in PBS at a concentration of 40.4 μg/ml, (average of 2.5 atoms of iodine per fragment), having about half the activity of the $^{131}$I-anti-CEA $F(ab')_2$ fragment and with $^{123}$I-Fab' instead of $^{123}$I-$F(ab')_2$ at a specific activity such that the proportional activity of I-131 to I-123 is the same as in Example 20(b).

(d) A sterile solution according to the procedure of part (b) except that the antibody is the $^{131}$I-anti-CSAp $F(ab')_2$ prepared according to Example 16, stored in 1% HSA in PBS at a concentration of 24.4 μg/ml and having comparable specific activity.

(e) A sterile solution according to the procedure of part (b) except that the antibody is the $^{131}$I-anti-CEA/CSAp hybrid $F(ab')_2$ prepared according to Example 17, stored in 1% HSA in PBS at a concentration of 24.4 μg/ml and having comparable specific activity.

(f) A sterile solution according to the procedure of part (b) except that it further contains $^{131}$I-anti-AFP $F(ab')_2$ and $^{131}$I-anti-HCG $F(ab')_2$, 6.1 μg/ml each, stored in 1% HSA in PBS at concentrations of 24.4 μg/ml each, and having comparable specific activity, an equal volume of each being used in place of one volume of 1% HSA in PBS in the procedure of part (a).

(g) A sterile solution according to the procedure of part (e) except that it further contains the $^{131}$I-anti-AFP/HCG $F(ab')_2$ hybrid prepared according to Example 17, stored in 1% HSA in PBS at concentration of 20.4 μg/ml and having comparable specific activity.

(h) A sterile solution according to the procedure of part (b) except that the antibody is the $^{131}$I-anti-CGA/GEA $F(ab')_2$ fragment prepared according to Example 17, stored in 1% HSA in PBS at a concentration of 24.4 μg/ml and having comparable specific activity.

(i) A sterile solution according to the procedure of part (b) except that the antibody is $^{131}$I-anti-CEA-$^{10}$B $F(ab')_2$ fragment prepared according to Example 19, having an average of 5 diazo-linked carborane residues and 1 atom of iodine per antibody molecule, and a specific activity of about 25 μCi/μg. The final solution contains 30.6 μg/ml of the antibody.

(j) A sterile solution according to the precedure of part (b) except that the antibody is the $^{131}$I-anti-CEA/CSAp-$^{10}$B hybrid $F(ab')_2$ prepared according to Example 19, having an average of 5 diazo-linked carborane residues and 1 atom of iodine per antibody molecule, and a specific activity of about 25 μCi/μg. The final solution contains 30.6 μg/ml of the antibody.

(k) A sterile solution according to the procedure of part (b) except that the antibody is the $^{131}$I-anti-AFP-$^{10}$B Fab' fragment prepared according to Example 19(d) having an average of 2 amide-linked carborane residues and 1 atom of iodine per antibody molecule, and a specific activity of about 52 μCi/μg. The final solution contains 16.8 μg/ml of the antibody.

(l) A sterile solution according to the procedure of part (b) except that the antibody is the $^{131}$I-anti-CGA/GEA-$^{10}$B hybrid $F(ab')_2$ fragment prepared according to Example 19(e), having an average of 5 diazo-linked carborane residues and 1 atom of iodine per antibody molecule, and a specific activity of about 25 μCi/μg. The final solution contains 30.6 μg/ml of the antibody.

Example 21
Tumor localization
Radioiodinated antibody fragment is administered to patients with suspected tumors. The patient is pretested for anaphylactic hypersensitivity to the corresponding IgG fragment. To block thyroid uptake of I-131 or I-123, Lugol's solution (Purepack) is administered by mouth, 5 drops twice daily for seven days beginning one day before injection of the radioactively labeled antibody fragment.

(a) Localization is effected according to the procedure of Goldenberg et al., *N. Eng. J. Med.,* 1384 (1978), by infusion of 3.5 ml of a solution of $^{131}$I-anti-CEA $F(ab')_2$ containing $^{123}$I-$F(ab')_2$ prepared according to Example 20(b) in 20 ml of sterile physiological saline over a period of from 10 minutes to 20 minutes. No Tc-99m compounds are used, the subtraction technique being adapted in a conventional fashion to discriminate between I-131 and I-123. Scans are taken immediately and at 2, 4, 8, 12, 24, 48 and 72 hours after injection of the fragment is completed.

Data analysis involves storing the photoscanning data in a computer, equalizing the activity level of the labeled normal IgG fragment with that of the labeled specific fragment in at least one non-target area and calculating a background level value for the labeled fragment for each data point; subtracting the resultant background value from the total fragment activity, pixel-by-pixel, to generate a value for the activity of targeted fragment for each data point; and using the resultant generated values for targeted fragment activity to generate a related output signal.

Significant localization is seen after 2 hours, with improved resolution with time, tending to plateau between 4 and 12 hours after injection. No additional background $^{123}$I-F(ab')$_2$ is added. The CEA-selectivity of this method is comparable to the earlier Goldenberg et al method, but the resolution, rapidity and convenience are enhanced significantly.

(b) The procedure of part (a) is followed using 3.5 ml of the solution of Example 20(c) instead of the solution of Example 20(b).

Imaging is comparable to that in part (a), being especially successful in patients with testicular and hepatic cancers. Secondary lung and abdominal metastases are well localized despite serum AFP levels which are often highly elevated.

(c) The procedure of part (a) is followed using 3.5 ml of the solution of Example 20(d) instead of the solution of Example 20(b).

Imaging is comparable to that in part (a), being especially successful in patients with colon cancers.

(d) The procedure of part (a) is followed using 3.5 ml of the solution of Example 20(e) instead of the solution of Example 20(b).

Imaging of gastrointestinal cancers is especially sharp, even compared to parts (a) and (c) of this Example.

(e) The procedure of part (a) is followed using 3.5 ml of the solution of Example 20(f) instead of the solution of Example 20(b).

Imaging of all tumors of the types successfully localized and detected in parts (a), (b) and (c) of this Example is successful. The combination scan gives enhanced localization and resolution in many cases, especially for testicular germ cell, liver and lung cancers.

(f) The procedure of part (a) is followed using 3.5 ml of the solution of Example 20(g) instead of the solution of Example 20(b).

Imaging is comparable to that in part (d) of this Example for all tumor types imaged by the procedures of parts (a)—(d) of this Example.

(g) The procedure of part (a) is followed using 3.5 ml of the solution of Example 20(h) instead of the solution of Example 20(b).

Imaging of a glioblastoma of the brain is successful, showing that the hybrid antibody can pass through the blood-brain barrier and localize in a brain tumor.

Example 22
Tumor therapy

(a) A patient having an ovarian cancer, optionally detected and localized by the procedure of Example 21, is injected by intravenous infusion with 150 mCi of the solution of Example 20(a) in 50 ml of sterile physiological saline. Reduction in tumor size is observed within 20 days. The dose is repeated at intervals adjusted on an individual basis.

(b) A patient having a cervical cancer optionally detected and localized by the procedure of Example 21, is injected with an amount of the solution of Example 20(i) (in 50 ml of sterile physiological saline) sufficient to provide 2.8 mCi of $^{131}$I activity based on a 70 kg patient weight.

The tumor is precisely localized 12 hours after injection using the procedure of Example 21. A well collimated beam of thermal neutrons is focused on the defined tumor locations. Irradiation with $3 \times 10^{12}$ neutrons per cm$^2$ is effected for each tumor locus, and is optionally repeated with administration of the tumor-localizing antibody, with or without the radiolabel, at intervals adjusted on an individual basis. Successful tumor reduction is observed.

(c) The procedure of part (b) is repeated except that the solution of Example 20(j) is used instead of the solution of Example 20(i), and the patient has a colon cancer. Successful tumor reduction is observed.

(d) The procedure of part (b) is repeated except that the solution of Example 20(k) is used instead of the solution of Example 20(i), and the patient has a germ-cell tumor of the testis. Successful tumor reduction is observed, including reduction of abdominal metastases.

(e) The procedure of part (b) is repeated except that the solution of Example 20(l) is used instead of the solution of Example 20(i), and the patient has a glioblastoma of the brain. Successful tumor reduction is observed.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

**Claims**

1. A F(ab')$_2$ hybrid formed from two chemically joined Fab' fragments, said fragments being a first marker-specific Fab' fragment obtained by cleavage of an antibody specific to a first tumor-associated marker and a second, different marker-specific Fab' fragment obtained by cleavage of a second antibody specific to the same or different tumor-associated marker, said hybrid being radiolabeled with a pharmacologically inert radio- isotope capable of detection using a photoscanning device.

2. The F(ab')₂ hybrid of claim 1 wherein said first marker-specific Fab' arm is obtained by cleavage of anti-CEA antibody, anti-AFP antibody, anti-HCG or HCG-beta antibody or anti-CSAp antibody; and especially wherein said second marker-specific Fab' arm is obtained by cleavage of a different antibody selected from anti-CEA antibody, anti-AFP antibody, anti-HCG or HCG-beta antibody and anti-CSAp antibody.

3. An injectable composition, comprising
(a) the F(ab')₂ hybrid of claim 1; and
(b) a pharmaceutically acceptable injection vehicle; for use in a method for detecting and localizing a tumor which produces or is associated with a cytoplasmic, intracellulor or cell-surface marker substance.

4. An antibody or antibody fragment which is specific to a marker substance produced by or associated with a tumor, said antibody or antibody fragment being radiolabeled with a pharmacologically inert radioisotope capable of detection with a photoscanning device, said labeled antibody or antibody fragment further containing in chemical combination an addend containing at least five atoms of boron with at least a natural abundance of Boron-10 isotope.

5. An injectable composition, comprising:
(a) the boron-containing, radiolabeled antibody or antibody fragment of claim 4; and
(b) a pharmaceutically acceptable injection vehicle; for use in a method of tumor radiotherapy.

6. An injectable composition, comprising:
(a) an antibody specific to a tumor cell-surface marker substance and radiolabeled with a pharmacologically inert radioisotope capable of detection using a photoscanning device;
(b) normal immunoglobulin from the same or different species as that used to prepare said specific antibody, said normal immunoglobulin being radiolabeled with a different pharmacologically inert radioisotope from that used to label the specific antibody and omitting at an energy capable of independent detection using said photoscanning device, said labeled normal immunoglobulin having substantially the same kinetics of binding, distribution and metabolism as the labeled specific antibody; and
(c) a pharmaceutically acceptable injection vehicle; for use in a method for determining the location of a tumor which produces or is associated with a cell-surface marker substance, using a subtraction technique.

7. An injectable composition of claim 6, wherein said cell-surface marker substance is CEA.

8. An injectable composition comprising:
(a) a substantially monospecific antibody, having a specific immunoreactivity of at least 70% to an intracellular marker substance produced by or associated with a tumor and a cross-reactivity to non-tumor associated antigens of less than 15% said antibody being radiolabeled with a pharmacologically inert radioisotope capable of detection using a photoscanning device;
(b) normal immunoglobulin from the same or different species as that used to prepare said specific antibody, said normal immunoglobulin being radiolabeled with a different pharmacologically inert radioisotope from that used to label the specific antibody and emitting at an energy capable of independent detection by said photoscanning device, said labeled normal immunoglobulin having substantially the same kinetics of binding distribution and metabolism as the labeled specific antibody; and
(c) a pharmaceutically acceptable injection vehicle; for use in a method for detecting and localizing a tumor which produces or is associated with an intracellular marker substance, using a subtraction technique.

9. An injectable composition, comprising:
(a) an antibody fragment specific to a tumor-associated cytoplasmic, intracellular or cell-surface marker, obtained by cleavage of an antibody specific to said marker substance, said fragment being radiolabeled with a pharmacologically inert radioisotope capable of detection using a photoscanning device; and
(b) a pharmaceutically acceptable injection vehicle; for use in a method for detecting and localizing a tumor which produces or is associated with a cytoplasmic, intracellular or cell-surface marker substance.

10. The antibody, antibody fragment or composition of any of claims 5, 8 and 9, wherein said marker substance or substances are selected from among carcinoembryonic antigen (CEA), alphafetoprotein (AFP), human chorionic gonadotropin (HCG) of the beta-subunit thereof, colon-specific antigen-p (CSAp), prostatic acid phosphatase, pancreatic oncofetal antigen, placental alkaline phosphatase, pregnancy beta₁-globulin, parathormone, calcitonin, tissue polypeptide antigen, T-antigen, beta₂-microglobulin, mammary tumor-associated glycoproteins (MTGP), galactosyl transferase-II (GT-II), gp-52 viral-associated antigen, ovarian cystadenocarcinoma-associated antigen (OCAA), ovarian tumor-specific antigen (OCA), cervical cancer antigens (CA-58, CCA, TA-4), basic fetoprotein (BFP), terminal deoxynucleotidyl transferase (TdT), cytoplasmic melanoma-associated antigens, human astrocytoma-associated antigen (HAAA), common glioma antigen (CGA), glioembryonic antigen (GEA), glial fibrillary acidic protein (GFA), common mengingioma antigen (CMA), ferritin, and tumor angiogenesis factor (TAF).

11. The injectable composition of any of claims 3, 5, 9 or 10, wherein said composition further comprises normal immunoglobulin or the corresponding fragment obtained by analogous cleavage of normal immunoglobulin from the same or different species as that used to prepare said specific antibody; said normal immunoglobulin being radiolabeled with a different pharmacologically inert radioisotope from that used to label the specific antibody and emitting at an energy capable of independent detection using said photoscanning device, said labeled normal immunoglobulin having substantially the same kinetics of binding, distribution and metabolism as the labeled specific antibody.

12. The injectable composition of any of claims 6—11, wherein the specific antibody or antibody fragment is labeled with one of a pair of radioisotopes, and the corresponding normal immunoglobulin or corresponding fragment is labeled with the other of the pair, said pair being one of Iodine-131 and Iodine-123; Indium-111 and Indium-113m; Gallium-67 and Gallium-68; Ruthenium-97 and Ruthenium-103; or Mercury-197 and Mercury-203.

**Patentansprüche**

1. Ein F(ab')$_2$-Hybrid, das aus zwei chemisch miteinander verbundenen Fab'-Fragmenten gebildet ist, von denen des eine ein erstes markerspezifisches Fab'-Fragment ist, welches durch Spaltung eines Antikörpers erhalten worden ist, der spezifisch gegenüber einem ersten tumorassoziierten Marker ist, und das andere ein zweites, unterschliedliches markerspezifisches Fab'-Fragment ist, welches durch Spaltung eines zweiten Antikörpers erhalten worden ist, der spezifisch gegenüber dem gleichen oder gegenüber einem anderen tumorassoziierten Marker ist, wobei dieses Hybrid radiomarkiert ist mit einem pharmakologisch inerten Radioisotop, das mittels einer Photoabtast-Vorrichtung nachgewiesen werden kann.

2. Das F(ab')$_2$-Hybrid gemäß Anspruch 1, dadurch gekennzeichnet, daß dieser erste markerspezifische Fab'-Arm erhalten worden ist durch Spaltung von anti-CEA-Antikörpern, anti-AFP-Antikörpern, anti-HCG- oder HCG-beta-Antikörpern oder anti-CSAp-Antikörpern, und daß dieser zweite markerspezifische Fab'-Arm erhalten worden ist durch Spaltung eines anderen Antikörpers aus der Gruppe, bestehend aus anti-CEA-Antikörpern, anti-AFP-Antikörpern, anti-HCG- oder HCG-beta-Antikörpern und anti-CSAp-Antikörpern.

3. Eine injizierbare Zusamensetzung, enthaltend

(a) das F(ab')$_2$-Hybrid gemäß Anspruch 1 und

(b) ein pharmazeutisch akzeptables Injektionsvehikel, zur Verwendung in einem Verfahren zum Nachweis und zum Lokalisieren eines Tumors, der assoziiert ist mit einer cytoplasmischen, intrazellulären oder Zelloberflächenmarker-Substanz oder diese Substanz produziert.

4. Ein Antikörper oder Antikörperfragment, der oder das spezifisch ist gegenüber einer Markersubstanz, welche von einem Tumor erzeugt wird oder mit diesem assoziiert ist, wobei dieser Antikörper oder dieses Antikörperfragment radiomarkiert ist mit einem pharmakologisch inerten Radioisotop, das mittels einer Photoabtast-Vorrichtung nachgewiesen werden kann, und wobei dieser markierte Antikörper oder das markierte Antikörperfragment weiterhin in chemischer Kombination einen Addenden enthält, welcher mindestens fünf Boratome enthält bei einem natürlichen Überwiegen des Bor-10-Isotops.

5. Eine injizierbare Zusammensetzung, enthaltend

(a) den borhaltigen, radiomarkierten Antikörper oder das Antikörperfragment gemäß Anspruch 4 und

(b) ein pharmazeutisch akzeptables Injektionsvehikel, zur Verwendung in einem Verfahren der Tumortherapie.

6. Eine injizierbare Zusammensetzung, enthaltend

(a) einen Antikörper, der spezifisch ist gegenüber einer Tumorzelloberflächenmarker-Substanz und der radiomarkiert ist mit einem pharmakologisch inerten Radioisotop, das mittels einer Photoabtast-Vorrichtung nachgewiesen werden kann.

(b) normales Immunglobulin von der gleichen oder einer anderen Spezies als der, die zur Gewinnung dieses spezifischen Antikörpers verwendet wurde, wobei dieses normale Immunglobulin radiomarkiert ist mit einem pharmakologisch inerten Radioisotop, das verschieden ist von dem, welches zur Markierung des spezifischen Antikörpers verwendet wurde und bei einer Energie emittiert, die einen unabhängigen Nachweis mittels einer Photoabtast-Vorrichtung erlaubt, und wobei dieses markierte normale immunglobulin im wesentlichen die gleiche Kinetik der Bindung, der Verteilung und des Stoffwechsels aufweist wie der markierte spezifische Antikörper, sowie

(c) ein pharmazeutisch akzeptables Injektionsvehikel, zur Verwendung in einem Verfahren zum Bestimmen des Ortes eines Tumors, welcher eine Zelloberflächenmarker-Substanz erzeugt oder mit dieser assoziiert ist, unter Anwendung einer Subtraktionstechnik.

7. Eine injizierbare Zusammensetzung gemäß Anspruch 6, dadurch gekennzeichnet, daß diese Zelloberflächenmarker-Substanz CEA ist.

8. Eine injizierbare Zusammensetzung, enthaltend

(a) einen im wesentlichen monospezifischen Antikörper, der eine spezifische Immunoreaktivität von mindestens 70% gegenüber einer intrazellulären Markersubstanz hat, die erzeugt wird von oder assoziiert ist mit einem Tumor, und eine Kreuzreaktivität von weniger als 15% gegenüber nichttumorassoziierten Antigenen, wobei dieser Antikörper radiomarkiert ist mit einem pharmakologisch inerten Ratioisotop, das mittels einer Photoabtast-Vorrichtung nachgewiesen werden kann.

(b) normales Immunglobulin von der gleichen oder einer anderen Spezies als der, die zur Erzeugung dieses spezifischen Antikörpers verwendet wurde, wobei dieses normale Immunglobulin radiomarkiert ist mit einem pharmakologisch inerten Radioisotop, das verschieden ist von dem, welches zur Markierung des spezifischen Antikörpers verwendet wurde, und das bei einer Energie emittiert, die einen unabhängigen Nachweis durch diese Photoabtast-Vorrichtung erlaubt, und wobei dieses markierte normale Immunglobulin im wesentlichen die gleiche Kinetik der Bindung, der Verteilung und des Stoffwechsels aufweist wie der markierte spezifische Antikörper, sowie

(c) ein pharmazeutisch akzeptables Injektionsvehikel, zur Verwendung in einem Verfahren zum Bestimmen und Lokalisieren eines Tumors, der eine intrazelluläre Markersubstanz erzeugt oder mit dieser assoziiert ist, unter Anwendung einer Subtraktionstechnik.

9. Eine injizierbare Zusammensetzung, enthaltend

(a) ein Antikörperfragment, das spezifisch ist gegenüber einem tumorassoziierten cytoplasmischen, intracellulären oder Zelloberflächenmarker, welcher erhalten worden ist durch Spaltung eines gegenüber dieser Markersubstanz spezifischen Antikörpers, wobei dieses Fragment radiomarkiert ist mit einem pharmakologisch inerten Radioisotop, das mittels einer Photoabtast-Vorrichtung nachgewiesen werden kann, und

(b) ein pharmazeutisch akzeptables Injektionsvehikel, zur Verwendung in einem Verfahren zum Nachweis und zum Lokalisieren eines Tumors, der eine cytoplastische, intrazelluläre oder Zelloberflächenmarker-Substanz erzeugt oder mit dieser assoziiert ist.

10. Der Antikörper, das Antikörperfragment oder eine Zusammensetzung gemäß einem der Ansprüche 5, 8 und 9, dadurch gekennzeichnet, daß diese Markersubstanz oder -substanzen ansgewählt ist oder sind aus der Gruppe, bestehend aus carcinoembryonischem Antigen (CEA), alpha-Fetoprotein (AFP), chorionischem Humangonadotropin (HCG) oder dessen beta-Untereinheit, colonspezifischem Antigen-p (CSAp), prostatischer Säurephosphatase, pankreatischem Oncofetal-Antigen, Placenta-Alkalinphosphatase, Schwangerschafts-$\beta_1$-Globulin, Parathormon, Calcitonin, Gewebe-polypeptid-Antigen, T-Antigen, beta$_2$-Mikroglobulin, Mammatumorassoziierten Glycoproteinen (MTGP), Galactosyltransferase-II (GT-II), gp-52 viralassoziiertem Antigen, Ovarcystadenocarcinoma-assoziiertem Antigen (OCAA), Ovartumorspezifischem Antigen (OCA), Cervical-Krebs-Antigenen (CA-58, CCA, TA-4), Basischem Fetoprotein (BFP), endständiger Deoxynucleotidyltransferase (TdT), cytoplasmischen Melanoma-assoziierten Antigenen, Astrocytoma-assoziiertem Human-Antigen (HAAA), Gewöhnlichem Glioma-Antigen (CGA), Glioembryonischem Antigen (GEA), glialfibrillärem saurem Protein (GFA), Gewöhnlichem Mengingioma-Antigen (CMA), Ferritin und Tumorangiogenesis-Faktor (TAF).

11. Injizierbare Zusammensetzung gemäß einem der Ansprüche 3, 5, 9 oder 10, dadurch gekennzeichnet, daß sie außerdem normales Immunglobulin oder das entsprechende Fragment enthält, welches erhalten wird durch analoge Spaltung von normalem Immunglobulin von der gleichen oder einer anderen Spezies als der, die zur Erzeugung dieses spezifischen Antikörpers verwendet wurde, wobei dieses normale Immunglobulin radiomarkiert ist mit einem Radioisotop, das verschieden ist von dem, welches zur Markierung des pharmakologisch inerten spezifischen Antikörpers verwendet wurde, und das bei einer Energie emittiert, die einen unabhängigen Nachweis mittels dieser Photoabtast-Vorrichtung erlaubt, und wobei dieses markierte normale Immunglobulin im wesentlichen die gleiche Kinetik der Bindung, der Verteilung und des Stoffwechsels aufweist wie der markierte spezifische Antikörper.

12. Injizierbare Zusammensetzung gemäß einem der Ansprüche 6 bis 11, dadurch gekennzeichnet, daß der spezifische Antikörper oder das Antikörperfragment markiert ist mit einem aus einem Paar von Radioisotopen, und daß das entsprechende normale Immunglobulin oder entsprechende Fragment markiert ist mit dem anderen aus diesem Paar, wobei dieses Paar besteht aus Jod-131 und Jod 123, Indium-111 und Indium-113m, Gallium-67 und Gallium-68, Ruthenium-97 und Ruthenium-103 oder Quecksilber-197 und Quecksilber-203.

## Revendications

1. Un hybride F(ab')$_2$ formé à partir de deux fragments Fab' unis chimiquement, lesdits fragments étant un premier fragment Fab' spécifique de marqueur obtenu par clivage d'un anticorps spécifique d'un premier marqueur associé à une tumeur et un second fragment Fab' spécifique de marqueur différent obtenu par clivage d'un second anticorps spécifique du même marqueur associé à une tumeur ou d'un marqueur différent, ledit hybdide étant radiomarqué avec un radioisotope pharmacologiquement inerte pouvant être détecté avec un dispositif de photoscintigraphie.

2. L'ybride F(ab')$_2$ de la revendication 1, dans lequel ledit premier bras Fab' spécifique de marqueur est obtenu par clivage d'un anticorps anti-CEA, d'un anticorps anti-AFP, d'un anticorps anti-HCG ou HCG-$\beta$ ou d'un anticorps anti-CSAp; et en particulier dans lequel ledit second bras Fab' spécifique de marqueur est obtenu par clivage d'un anticorps différent choisi parmi un anticorps anti-CEA, un anticorps anti-AFP, un anticorps anti-HCG ou HCG-$\beta$ et un anticorps anti-CSAp.

3. Une composition injectable comprenant

(a) l'hybride F(ab')$_2$ de la revendication 1; et

(b) un véhicule d'injection convenant en pharmacie; pour l'emploi dans un procédé pour la détection et la localisation d'une tumeur qui produit, ou est associée à, une substance de marquage cytoplasmique, intracellulaire ou de surface cellulaire.

4. Un anticorps ou un fragment d'anticorps qui est spécifique d'une substance de marquage produite par, ou associée à, une tumeur, ledit anticorps ou fragment d'anticorps étant radiomarqué avec un radio-isotope pharmacologiquement inerte pouvant être détecté avec un dispositif photoscintigraphique, ledit anticorps ou fragment d'anticorps marqué contenant de plus en combinaison chimique un composé fixé par addition contenant au moins 5 atomes de bore avec au moins l'abondance naturelle de l'isotope bore 10.

5. Une composition injectable comprenant

(a) l'anticorps ou le fragment d'anticorps radiomarqué contenant du bore de la revendication 4 et

(b) un véhicule d'injection convenant en pharmacie; pour l'emploi dans un procédé de radiothérapie tumorale.

6. Une composition injectable comprenant

(a) un anticorps spécifique d'une substance de marquage de surface de cellules tumorales et radiomarqué avec un radio-isotope pharmacologiquement inerte pouvant être détecté avec un dispositif photoscintigraphique;

(b) une immunoglobuline normale de la même espèce que celle utilisée pour préparer ledit anticorps spécifique ou d'une espèce différente, ladite immunoglobuline normale étant radiomarquée avec un radio-isotope pharmacologiquement inerte différent de celui utilisé pour marquer l'anticorps spécifique et émettant une énergie pouvant être indépendamment détectée avec ledit dispositif photoscintigraphique, ladite immunoglobuline normale marquée ayant essentiellement les mêmes cinétiques de fixation, de distribution et de métabolisme que l'anticorps spécifique marqué; et

(c) un véhicule d'injection convenant en pharmacie; pour l'emploi dans un procédé de détermination de l'emplacement d'une tumeur qui produit, ou est associée à, une substance de marquage de surface cellulaire en utilisant une technique soustractive.

7. Une composition injectable de la revendication 6, dans laquelle ladite substance de marquage de surface cellulaire est le CEA.

8. Une composition injectable comprenant

(a) un anticorps essentiellement mono-spécifique ayant une immunoréactivité spécifique d'au moins 70% vis-à-vis d'une substance de marquage intracellulaire produite par, ou associée à, une tumeur et une réactivité croisée, pour les antigènes non associés à la tumeur, inférieure à 15%, ledit anticorps étant radiomarqué avec un radioisotope pharmacologiquement inerte pouvant être détecté avec un dispositif photoscintigraphique;

(b) une immunoglobuline normale de la même espèce que celle utilisée pour préparer ledit anticorps spécifique ou d'une espèce différente, ladite immunoglobuline normale étant radiomarquée par un radio-isotope pharmacologiquement inerte différent de celui utilisé pour marquer l'anticorps spécifique et émettant une énergie pouvant être indépendamment détectée avec ledit dispositif photoscintigraphique, ladite immunoglobuline normale marquée ayant essentiellement les mêmes cinétiques de fixation, de distribution et de métabolisme que l'anticorps spécifique marqué; et

(c) un véhicule d'injection acceptable en pharmacie; pour l'emploi dans un procédé de détection et de localisation d'une tumeur qui produit, ou est associée à une substance de marquage intracellulaire, en utilisant une technique soustractive.

9. Une composition injectable comprenant

(a) un fragment d'anticorps spécifique d'un marqueur cytoplasmique, intracellulaire ou de surface cellulaire associé à une tumeur, obtenu par clivage d'un anticorps spécifique de ladite substance de marquage, ledit fragment étant radiomarqué avec un ratio-isotope pharmacologiquement inerte pouvant être détecté avec un dispositif photoscintigraphique; et

(b) un véhicule d'injection convenant en pharmacie; pour l'emploi dans un procédé de détection et de localisation d'une tumeur qui produit, ou est associée à, une une substance de marquage cytoplasmique, intracellulaire ou de surface cellulaire.

10. L'anticorps, le fragment d'anticorps ou la composition de l'une quelconque des revendications 5, 8 et 9, où ladite substance ou lesdites substances de marquage sont choisies parmi l'antigène carcinoembryonnaire (CEA), l'α-foeto-protéine (AFP), la gonadotrophine chorionique humaine (HCG) ou sa sous-unité β, l'antigène p spécifique du côlon (CSAp), la phosphatase acide prostatique, l'antigène oncofoetal pancréatique, la phosphatase alcaline placentaire, la $\beta_1$-globuline de la grossesse, la parathormone, la calcitonine, l'antigène polypeptidique tissulaire, l'antigène T, la microglobuline $\beta_2$, les glyco-protéines associées à une tumeur mammaire (MTGP), la galactosyl transférase-II (GT-II), l'antigène associé au virus gp-52, l'antigène associé au cystadénocarcinome ovarien (OCAA), l'angigène spécifique des tumeurs ovariennes (OCA), les antigènes spécifiques du cancer du col (CA-58, CCA, TA-4), la foetoprotéine basique (BFP), la désoxynucléotidyl transférase terminale (TdT), les antigènes cytoplasmiques associés au mélanome, l'antigène associé à l'astrocytome humain (HAAA), l'antigène commun du gliome (CGA), l'antigène glioembryonnaire (GEA), la protéine acide fibrillaire gliale (GFA), l'antigène commun du méningiome (CMA), la ferritine et le facteur d'angiogenèse tumorale (TAF).

11. La composition injectable de l'une quelconque des revendications 3, 5, 9 ou 10, dans laquelle ladite composition comprend de plus une immunoglobuline normale ou le fragment correspondant obtenu par clivage analogue d'une immunoglobuline normale de la même espèce que celle utilisée pour préparer ledit anticorps spécifique ou d'une espèce différente; ladite immunoglobuline normale étant radiomarquée avec un radio-isotope pharmacologiquement inerte différent de celui utilisé pour marquer l'anticorps spécifique et émettant une énergie pouvant être détectée indépendamment avec ledit dispositif photoscintigraphique, ladite immunoglobuline normale marquée ayant essentiellement les mêmes cinétiques de fixation, de distribution et de métabolisme que l'anticorps spécifique marqué.

12. La composition injectable de l'une quelconque des revendications 6 à 11, dans laquelle l'anticorps ou le fragment d'anticorps spécifiques est marqué avec un des radio-isotopes d'une paire et l'immunoglobuline normale correspondante ou le fragment correspondant est marqué avec l'autre radio-isotope de la paire, ladite paire étant l'une de l'iode 131 et l'iode 123, l'indium 111 et l'indium 113m, le gallium 67 et le gallium 68, le ruthénium 97 et le ruthénium 103 ou le mercure 197 et le mercure 203.